# EUROPEAN PATENT APPLICATION

(11) **EP 3 533 878 A1**
(43) Date of publication of application: **04.09.2019**
(21) Application number: 18159212.2
(22) Date of filing: 28.02.2018
(51) Int. Cl.: C12P 7/44, C12N 1/14, C12N 9/10, C12N 9/88

(54) **PROCESS FOR PRODUCING CITRAMALIC ACID EMPLOYING ASPERGILLUS**

(71) Applicant: Dutch DNA Biotech B.V., 3584 CH Utrecht (NL)
(72) Inventor: Punt, Peter Jan, 3584 CH Utrecht (NL); Hossain, Abeer Hossain, 3584 CH Utrecht (NL)
(74) Representative: V.O.

(57) **Abstract**

The invention relates to a method to increase production of of citramalic acid in a micro-organism by overexpression of itaconyl-CoA transferase (EC 2.8.3.-), itaconyl-CoA hydratase (citramalyl-CoA hydro-lyase; EC4.2.1.56), and/or citramalate synthase (EC 2.3.1.182) and/or inhibiting the expression or functioning of the enzyme citramalate dehydratase (EC4.2.1.34) or the enzyme citramalate lyase (EC4.1.3.22), or by an increase in the level of cytosolic acetyl-CoA. Also embodied are micro-organisms, preferably *Aspergillis terreus* or *Aspergillus niger,* in which said enzyme(s) is/are expressed and/or silenced.

## Description

The invention relates to the field of microbial production, more specifically production of organic acids, such as citramalic acid (citramalate) and itaconic acid (itaconate), more specifically the production thereof in micro-organisms.

Organic acid production in micro-organisms is one of the industrial processes that has been extensively studied in the past decennia. Basis for the production is the citric acid cycle, or Krebs cycle, which is one of the most fundamental and ubiquitous metabolic pathways in living organisms. In eukaryotes this process takes place in the mitochondrion and is fed mainly by pyruvate and acetyl-CoA that are transported from the cytoplasm into the mitochondrion.

Because it is a main starting ingredient for the production of plastics, the production and metabolism of itaconic acid in microbial cells has been one of the major objects of these studies (Calam, C.T. et al., 1939, Thom. J. Biochem., 33:1488-1495; Bentley, R. and Thiessen, C.P., 1956, J. Biol. Chem. 226:673-720; Cooper, R.A. and Kornberg, H.L., 1964, Biochem. J., 91:82-91; Bonnarme, P. et al., 1995, J. Bacteriol. 117:3573-3578; Dwiarti, L. et al., 2002, J. Biosci. Bioeng. 1:29-33). However, the metabolic pathway for itaconic acid has not been unequivocally established (Wilke, Th. and Vorlop, K.-D., 2001, Appl. Microbiol. Biotechnol. 56:289-295; Bonnarme, P. et al., 1995, J. Bacteriol. 177:3573-3578). Two complicating factors in this respect are that the biosynthesis route for itaconic acid is thought to occur both in the cytosol and the mitochondria (Jaklitsch, W.M. et al., 1991, J. Gen. Microbiol. Appl. 6:51-61) and that aconitase, the enzyme that interconverts citric acid into cis-aconitate, and vice versa, and other enzymes in the metabolic pathway have been found to be present in many isoforms in microbial cells.

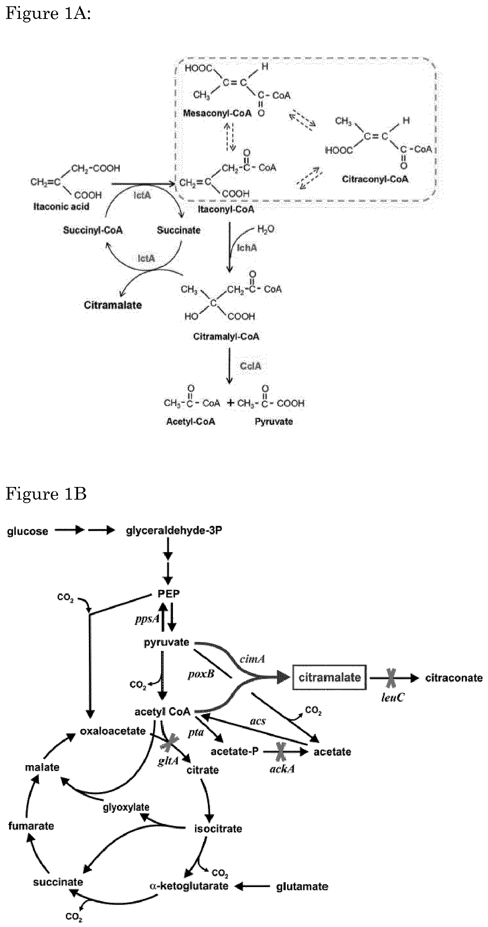

The general scheme currently envisioned for itaconic acid biosynthesis is given in Fig. 1, wherein clearly the existence of the biosynthetic route both in the cytosol and the mitochondria is depicted and the putative connection between these two compartments. At several point of this scheme possibilities exist to try to improve the existing commercial production of itaconic acid in micro-organisms. The production of itaconic acid from citrate has been achieved in Aspergillus (and also other micro-organisms) with technology as described in WO 2009/014437, WO 2009/104958 and WO 2009/110796.

Citramalate, a chemical precursor to the industrially important methacrylic acid (MAA), can be synthesized using *Escherichia coli* overexpressing citramalate synthase (cimA gene) (Wu X, Eiteman MA, Biotechnol. Bioeng. 113:2670-5, 2016). It was recently found that *E. coli* MG1655 gltA leuC ackA/pZE12-cimA containing three key knockouts (citrate synthase, 3-isopropylmalate dehydratase and acetate kinase) accumulated 46 g/L citramalate from glucose at a yield of 0.63 g/g (75% of the theoretical maximum). However, 10 g/L acetate also accumulated despite the deletion of acetate kinase. Acetate formation is undesirable because this acid negatively impacts cell growth even at concentrations as low as 0.5 g/L; further, it is a sink which diverts carbon that could otherwise be used to synthesize the desired product. Knocking out the ackA-pta and poxB genes, coding for the two major acetate production pathways in *E. coli* was found to overcome the undesired formation of excess acetate (Parimi, N.S. et al., Microb. Cell factories, 16:114, 2017)

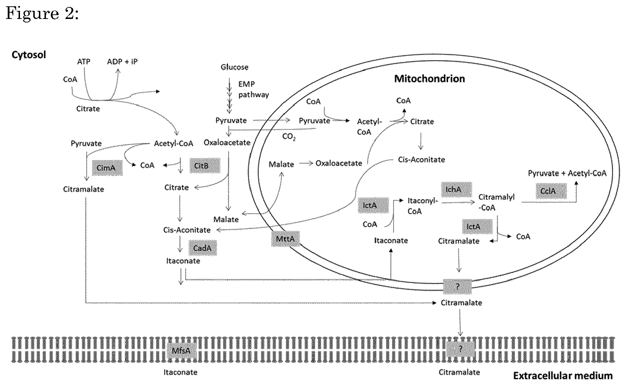

Next to the problem of the by production of acetate it is still unknown, as is depicted in Fig. 2, in what respect the production of citramalate takes place in- or outside of the mitochondrion. There is still a need to know more about the production of citramalate and to provide for an improved synthesis of this molecule.

Because of this improved and/or alternative production systems for citramalate are strongly desired.

### SUMMARY OF THE INVENTION

The present inventors now have found mutant strains producing improved levels of organic acids in general and more specifically improved levels of itaconic acid and citramalic acid. Moreover the present inventors have found two different biosynthetic pathways, one converting acetyl-CoA and pyruvate to citramalic acid directly via citramalate synthase / isopropyl malate synthase IPMS (EC 2.3.1.182) and one converting itaconic acid to citramalic acid via itaconyl-CoA transferase (EC 2.8.3.-) and itaconyl-CoA hydratase / citramalyl-CoA hydro-lyase (EC 4.2.1.56) from the organism in which itaconic acid is produced, e.g. *Aspergillus* which are able to boost the production of citramalic acid when overexpressed. In addition, the inventors have found mutant strains producing improved levels or organic acids, specifically itaconic acid and citramalic acid in such acid producing strains by overexpression of ATP-citrate lyase (EC 2.3.3.8), producing a precursor acetyl-CoA for organic acid production. Accordingly, the invention comprises methods to increase production of organic acids in general and more specifically itaconic acid and/or citramalic acid in a micro-organism.

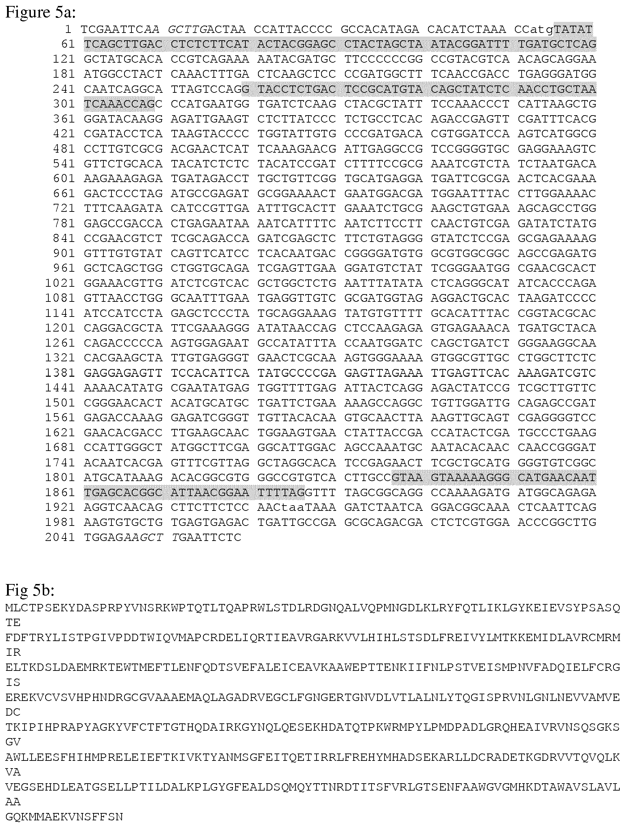

More particularly, the inventors have identified a new method to increase production of citramalic acid in a micro-organism by overexpression of itaconyl-CoA transferase (EC 2.8.3.-), itaconyl-CoA hydratase (citramalyl-CoA hydro-lyase; EC4.2.1.56), and/or citramalate synthase (EC 2.3.1.182). Preferred in said method is wherein the itaconyl-CoA transferase is ATEG_06299 or An07g00760 or an ortholog thereof. Also preferred in said method is wherein the itaconyl-CoA hydratase is ATEG_03709 of An07g09220 or an ortholog thereof. Further, also preferred in said method is wherein the citramalate synthase is An09g00170 or a protein that has a sequence identity of 60% with the sequence as depicted in Fig. 5b or an ortholog thereof, more preferably wherein said protein is coded by a nucleotide sequence as depicted in Fig, 5a.

Also part of the invention is a method to increase production of citramalic acid by inhibition of the citramalic acid pathway, in particular by inhibition of the enzyme citramalate dehydratase (EC4.2.1.34) or the enzyme citramalate lyase (EC4.1.3.22), or by an increase in the level of cytosolic acetyl-CoA. In a preferred embodiment of this invention the citramalate dehydratase is chosen from aconitate hydratase (e.g. An08g10530, An09g03870, An02g11040, An16g05760), aconitase (e.g. An15g07730), homoaconitate hydratase (e.g. An15g00350) and isopropylmalate dhydratase (e.g. An02g03250). In a further preferred embodiment of this invention the citramalate lyase is chosen from oxaloacetate acetylhydrolase (e.g. An07g08390), oxaloacetate hydrolase (e.g. An11g07720), methyl-isocitrate lyase (e.g. An12g07630), isocitrate lyase (e.g. An01g09270), malate synthase (e.g. An15g02980, An12g05180). In a further preferred embodiment of this invention the increase of level of cytosolic acetyl-CoA is caused by overexpression of ATP-citrate lyase, more particularly An11g00510 and/or An11g00530.

The invention further has as a preferred embodiment production in a micro-organism which naturally produces itaconic acid or citramalic acid,.
In another preferred embodiment said micro-organism is genetically constructed to produce citramalic acid, preferably by introducing a gene coding for itaconyl-CoA transferase (EC 2.8.3.-), itaconyl-CoA hydratase (citramalyl-CoA hydro-lyase; EC4.2.1.56), and/or citramalate synthase (EC 2.3.1.182). Further, it is preferred that said micro-organism is an *Aspergillus,* preferably *A*. *terreus* or *A*. *niger.*
Further part of the invention is a micro-organism, preferably *Aspergillus,* more preferably *A*. *terreus* or *A. niger,* in which expression of the gene(s) coding for the enzyme citramalate dehydratase (EC4.2.1.34) or the enzyme citramalate lyase (EC4.1.3.22) is inhibited. Further preferred is a micro-organism, preferably *Aspergillus,* more preferably *A*. *terreus* or *A*. *niger,* in which the gene(s) coding for the enzyme citramalate synthase (EC 2.3.1.182) is overexpressed
Another part of the invention is formed by mutant strains as deposited on 26-01-2018 under no. CBS 143860, CBS 143861 and CBS 143863 with the Westerdijk Institute in Utrecht (The Netherlands)

### LEGENDS TO THE FIGURES

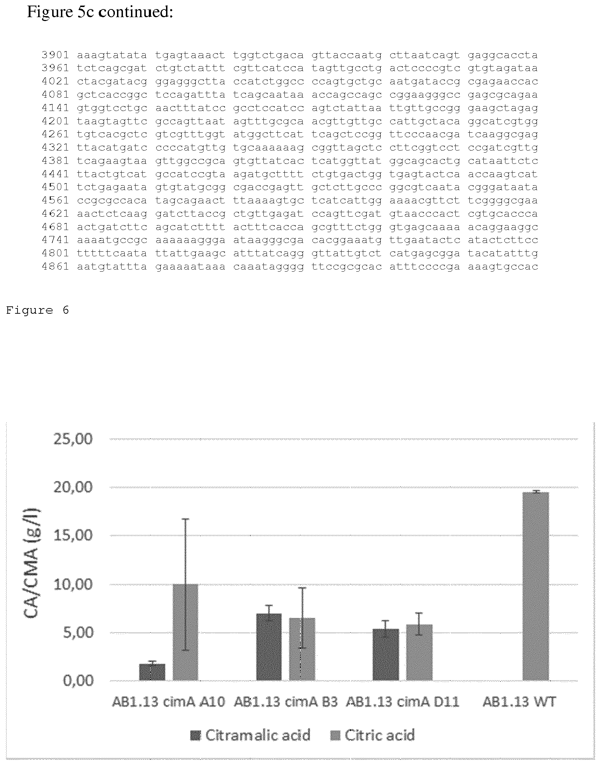

Figure 1
   A: itaconic acid bioconversion pathway (taken from Chen et al. 2016 Appl. Microbiol. Biotechnol. 100(17):7541-8)
   B: citramalic acid biosynthesis pathway (taken from Wu & Eiteman 2016 Biotechnol. Bioeng. 113(12):2670-2675)
Figure 2: citramalic acid biosynthesis routes in *Aspergillus niger* organic acid pathway
Fig. 3: Itaconic acid production and degradation, citramalic acid production and glucose consumption in high IA producing *Aspergillus niger* strains CitB #99 (CBS 141659) and EE #25 (CBS 141661) (A) and AB1.13 CCM B5 (CBS 143860) (B) incubated in non-shaking flasks. Both strains were cultivated in 500 mL erlenmeyers filled with 100 mL M12++ medium. Flasks were inoculated with 1.0*10⁶ spores/mL and incubated at 33°C in an incubator at 0 RPM. Itaconic acid (IA) titer (g/l), citramalic acid (CMA) titer (g/l), and glucose concentration (g/l) are shown in relation to incubation time (h).
Figure 4: Inhibition of itaconic acid biodegradation by deletion of ictA and ichA. Flask experiment with CitB99, CitB99 ΔICT and CitB99 ΔICH. The flask experiments were performed in duplo. (A) IA production of strains CitB99 ΔICT, CitB99 ΔICH and CitB99 in standing flask experiment. (B) Glucose consumption of strains CitB99 ΔICT, CitB99 ΔICH and CitB99.
Fig. 5 sequences of cimA.
5a: In vitro synthesized gene sequence of An09g00170 (cimA)by GeneArt, Hindlll restriction site in italics, Intron sequences are grey shaded, atg Start codon in small caps, taa Stop codon in small caps.
5b: translated AA sequence of An09g00170 (cimA)
5c: Sequence of the pABgpd-I expression vector. The Hindlll-Hindlll cloning sites used for insertion of the cimA gene to be overexpressed are indicated by shading
Fig. 6: Citramalic acid production in *cimA* transformants. *cimA* transformant strains AB1.13 CimA A10, B3, and D11 were incubated in 500 mL shake flasks with 100 mL M12+Cu medium. After 288h of incubation the flasks were harvested and citramalate concentration measured using HPLC.

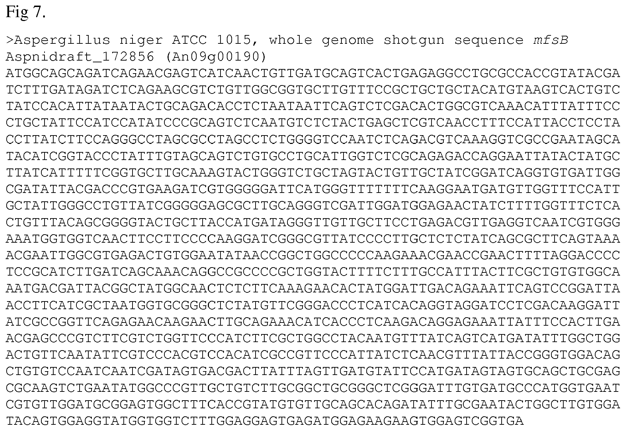

Fig 7; MfsB (An0900190) gene sequence, *Aspergillus niger* ATCC 1015, whole genome shotgun sequence mfsB Aspnidraft_172856 (An09g00190)

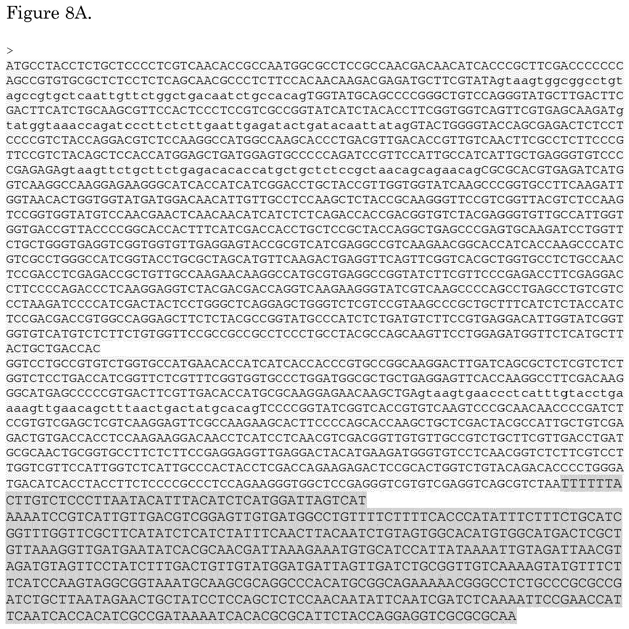

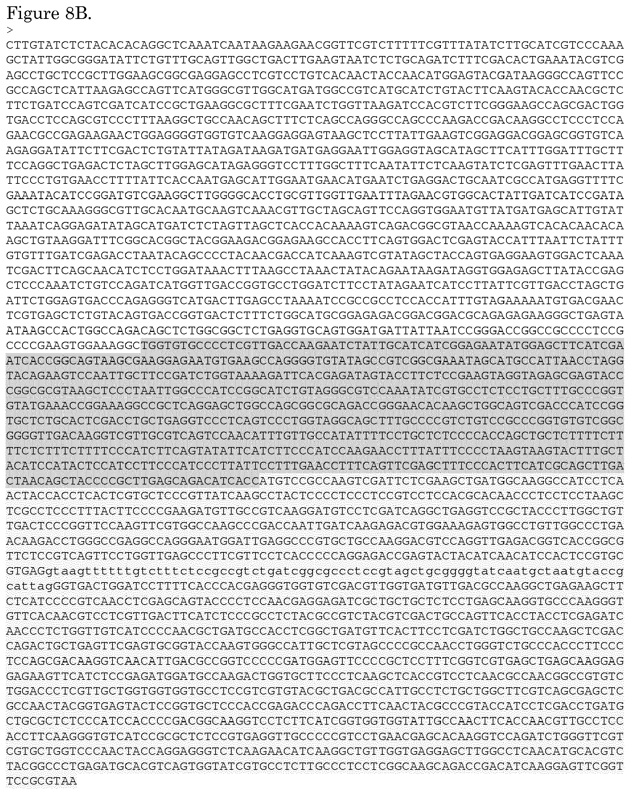

Figure 8 ATP-citrate lyase genes sequences cloned in pABgpd1; (A) ATP citrate lyase subunit 1 Aspergillus niger ATCC 1015 acl1; (B) ATP citrate lyase subunit 2 *Aspergillus niger* ATCC 1015 acl2,

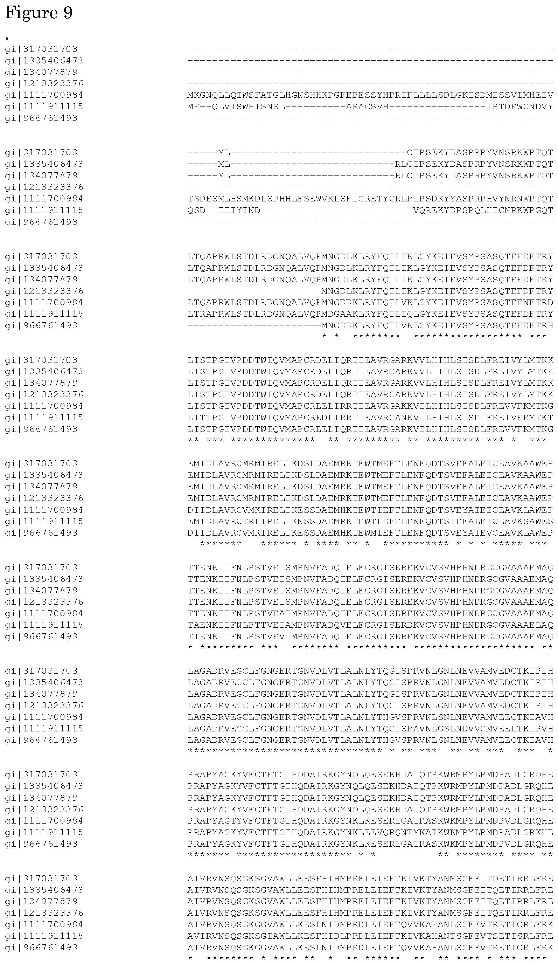

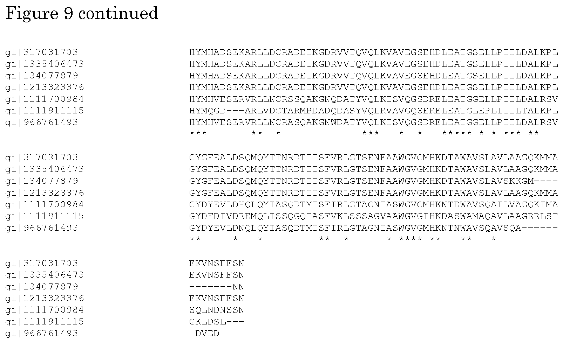

Fig. 9. Alignments of citramalate synthase cimA: XP_001393342.2 2-isopropylmalate synthase *[Aspergillus niger* CBS 513.88], SPB42959.1 unnamed protein product *[Aspergillus niger],* CAK40105.1 unnamed protein product *[Aspergillus niger],* OWW32599.1 HMGL-like family protein *[Aspergillus niger],* OJI87914.1 hypothetical protein ASPTUDRAFT_921801 *[Aspergillus tubingensis* CBS 134.48], OJJ76682.1 hypothetical protein ASPBRDRAFT_52246 [*Aspergillus brasiliensis* CBS 101740], GAQ45761.1 unnamed protein product [*Aspergillus niger];*

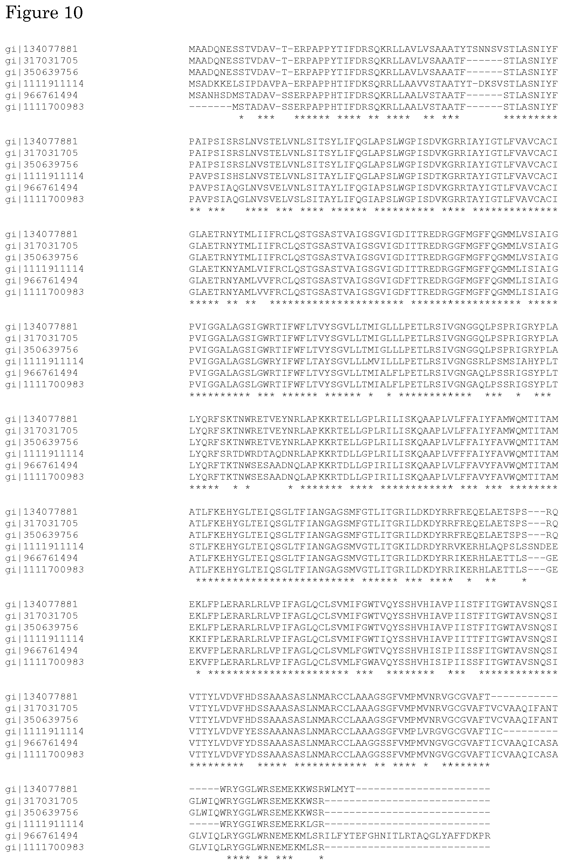

Figure 10. Alignments exporter mfsB: CAK40107.1 unnamed protein product [*Aspergillus niger*]*,* XP_001393344.2 MFS multidrug transporter [*Aspergillus niger* CBS 513.88], EHA28109.1 hypothetical protein ASPNIDRAFT_188577 [*Aspergillus niger* ATCC 1015], OJJ76681.1 hypothetical protein ASPBRDRAFT_116971 [*Aspergillus brasiliensis* CBS 101740], GAQ45762.1 hypothetical protein ASPNIDRAFT_188577 *[Aspergillus niger],* OJI87913.1 hypothetical protein ASPTUDRAFT_137059 [*Aspergillus tubingensis* CBS 134.48].

### DETAILED DESCRIPTION OF THE INVENTION

"Fungi" are herein defined as eukaryotic micro-organisms and include all species of the subdivision Eumycotina (Alexopoulos, C. J., 1962, In: Introductory Mycology, John Wiley & Sons, Inc., New York). The term fungus thus includes both filamentous fungi and yeast. "Filamentous fungi" are herein defined as eukaryotic micro-organisms that include all filamentous forms of the subdivision Eumycotina. These fungi are characterized by a vegetative mycelium composed of chitin, cellulose, and other complex polysaccharides. The filamentous fungi used in the present invention are morphologically, physiologically, and genetically distinct from yeasts. Vegetative growth by filamentous fungi is by hyphal elongation and carbon catabolism of most filamentous fungi are obligately aerobic. "Yeasts" are herein defined as eukaryotic micro-organisms and include all species of the subdivision Eumycotina that predominantly grow in unicellular form. Yeasts may either grow by budding of a unicellular thallus or may grow by fission of the organism.

The term "fungal", when referring to a protein or nucleic acid molecule thus means a protein or nucleic acid whose amino acid or nucleotide sequence, respectively, naturally occurs in a fungus.

The term "gene", as used herein, refers to a nucleic acid sequence containing a template for a nucleic acid polymerase, in eukaryotes, RNA polymerase II. Genes are transcribed into mRNAs that are then translated into protein.

"Expression" refers to the transcription of a gene into structural RNA (rRNA, tRNA) or messenger RNA (mRNA) with subsequent translation into a protein.

The term "inhibition" can be both used for inhibition of expression of a protein or for inhibition of function of a protein. When used in conjunction with protein expression the term "inhibition" refers to a measurable reduction in expression of mRNA encoding said protein or in the concentration of the protein in the cell. The reduction can be anything from less than normal to zero (i.e. no mRNA or protein measurable). Further, with respect to protein function, the term "inhibition" refers to any action and/or treatment which operates against the full activity of a protein thus reducing and/or completely suppressing protein function.

The term "vector" as used herein, includes reference to an autosomal expression vector and to an integration vector used for integration into the chromosome.

The term "expression vector" refers to a DNA molecule, linear or circular, that comprises a segment encoding a polypeptide of interest under the control of (i.e., operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular an expression vector comprises a nucleotide sequence that comprises in the 5' to 3' direction and operably linked: (a) a fungal-recognized transcription and translation initiation region, (b) a coding sequence for a polypeptide of interest, and (c) a fungal-recognized transcription and translation termination region. "Plasmid" refers to autonomously replicating extrachromosomal DNA which is not integrated into a microorganism's genome and is usually circular in nature.

An "integration vector" refers to a DNA molecule, linear or circular, that can be incorporated in a microorganism's genome and provides for stable inheritance of a gene encoding a polypeptide of interest. The integration vector generally comprises one or more segments comprising a gene sequence encoding a polypeptide of interest under the control of (i.e., operably linked to) additional nucleic acid segments that provide for its transcription. Such additional segments may include promoter and terminator sequences, and one or more segments that drive the incorporation of the gene of interest into the genome of the target cell, usually by the process of homologous recombination. Typically, the integration vector will be one which can be transferred into the host cell, but which has a replicon that is non-functional in that organism. Integration of the segment comprising the gene of interest may be selected if an appropriate marker is included within that segment.

"Transformation" and "transforming", as used herein, refer to the insertion of an exogenous polynucleotide into a host cell, irrespective of the method used for the insertion, for example, direct uptake, transduction, f-mating or electroporation. The exogenous polynucleotide may be maintained as a non-integrated vector, for example, a plasmid, or alternatively, may be integrated into the host cell genome.

By "host cell" is meant a cell that contains a vector or recombinant nucleic acid molecule and supports the replication and/or expression of the vector or recombinant nucleic acid molecule. Host cells may be prokaryotic cells such as *E. coli,* or eukaryotic cells such as yeast, fungus, plant, insect, amphibian, or mammalian cells. Preferably, host cells are fungal cells.

-Orthologs are homologous sequences descended from the same ancestral sequence for which function of the gene or sequence has been conserved across evolutionary time. According to another definition orthologs are genes in different species that have evolved from common ancestral gene by speciation and that retain the same or similar functions. For the presen invention orthologs may be considered as sequences with a certain degree of identity that have the same or similar function.

### Deposit information (see also Table 1)

Several of the strains that have been used or produced with the present invention have been deposited according to the following specifics. Mutant strains no. CBS 141653, CBS 141655, CBS 141657, CBS 141659, CBS 141660, CBS 141661 and CBS 141662 have been deposited on 22-07-2016 with the Westerdijk Institute in Utrecht (The Netherlands). Mutant strains no. CBS 143054, CBS 143055 and CBS 143056 have been deposited on 24-07-2017 with the Westerdijk Institute in Utrecht (The Netherlands). Further the mutant strains no. CBS 143860, CBS 143861 and CBS 143863 have been deposited on 26-01-2018 with the Westerdijk Institute in Utrecht (The Netherlands).

The present invention relates to the field of microbial production of itaconic acid and citramalic acid in fungi. The invention comprises results showing increased citramalic acid levels by biochemical conversion of itaconic acid or direct synthesis of citramalic acid, by
(1) improving itaconyl CoA transferase, itaconyl CoA hydratase (citramalyl-CoA hydro-lyase) activity from the production host.
(2) reducing /removing expression of citramalyl CoA lyase activity from the production host
(3) improving citramalate synthase activity from the production host
(4) improving citramalic acid export
(5) reducing/removing citramalate degradation from the production host
Additionally, the invention comprises results showing increased itaconic and/or citramalic acid levels by increasing levels of acetyl-CoA, a precursor for organic aicds by the biochemical conversion of citric acid to acetyl-CoA by improving ATP-citrate lyase activity from the production host.

The most direct biosynthesis route for citramalic acid involves citramalate synthase / isopropyl malate synthase IPMS (EC 2.3.1.182) converting acetyl-CoA and pyruvate directly to citramalic acid. Besides this citramalate synthase route also the enzymes itaconyl-CoA transferase and itaconyl-CoA hydratase (citramalyl-CoA hydro-lyase) are important enzymes in the biosynthesis of citramalic acid (see Fig. 2), they either convert itaconic acid via itaconyl-CoA into citramalyl-CoA, which is further converted by again itaconyl-CoA transferase into citramalic acid

The present invention discloses that overexpression of one or more of these enzymes improves production of citramalic acid in micro-organisms.

Overexpression of the gene encoding citramalate synthase would lead to increased levels of citramalic acid.

Preferably the citramalate synthase is a fungal protein, more preferably An09g00170 or a protein that has a sequence identity of at least 60% with the sequence as depicted in Fig. 5b or an ortholog thereof. More preferably, said protein has an identity of at least 70%, more preferably at least 75%, more preferably at least 80%, more preferably at least 85%, more preferably at least 90%, more preferably at least 95%, more preferably at least 96%, more preferably at least 97%, more preferably at least 98%, more preferably at least 99% identity with the amino acid sequence of An09g00170 as depicted in Fig. 5b.. Some of the orthologs of this protein are depicted in Fig. 9 and also proteins that have a sequence identity of at least 60% with the sequence as depicted in Fig. 5b or an ortholog thereof can be used in the present invention. More preferably, said protein has an identity of at least 70%, more preferably at least 75%, more preferably at least 80%, more preferably at least 85%, more preferably at least 90%, more preferably at least 95%, more preferably at least 96%, more preferably at least 97%, more preferably at least 98%, more preferably at least 99% identity with the amino acid sequences as depicted in Fig. 9.

In principle, also the overexpression of two of the three genes involved in the conversion of itaconic acid to acetyl-CoA and pyruvate (i.e. those encoding the enzymes itaconyl-CoA transferase and itaconyl-CoA hydratase) would be sufficient to produce citramalic acid. It is however advantageous to also block the step from citramalyl-CoA to pyruvate and acetyl-CoA mediated by citramalyl-CoA pyruvate lyase (the third step of the itaconic acid degradation pathway, if only as a precautious safety measure. Orthologs of the enzymes itaconyl-CoA-transferase, itaconyl-CoA-hydratase and citramalyl-CoA lyase have been disclosed in the co-pending application PCT/EP2017/071466 in figures 11, 12 and 13, respectively. It is believed that these genes or amino acid sequences that have a sequence identity of at least 60% with the sequence as depicted in Fig. 5b or an ortholog thereof. More preferably, said protein has an identity of at least 70%, more preferably at least 75%, more preferably at least 80%, more preferably at least 85%, more preferably at least 90%, more preferably at least 95%, more preferably at least 96%, more preferably at least 97%, more preferably at least 98%, more preferably at least 99% identity with the amino acid sequences of the Figs. 11, 12 and 13 of PCT/EP2017/071466 would be able to function in the present invention.

Next to these enzymes, it would also be possible to additionally overexpress the gene encoding a citramalic acid cellular exporter clustered with the citramalate synthase gene.Orthologs of this gene are shown in Fig. 10 and also proteins that have a sequence identity of at least 60% with the sequence as depicted in Fig. 5b or an ortholog thereof can be used in the present invention. More preferably, said protein has an identity of at least 70%, more preferably at least 75%, more preferably at least 80%, more preferably at least 85%, more preferably at least 90%, more preferably at least 95%, more preferably at least 96%, more preferably at least 97%, more preferably at least 98%, more preferably at least 99% identity with the amino acid sequences as depicted in Fig. 10.

Additionally, overexpression of cytosolic ATP-citrate lyase will lead to improved acetyl-CoA (produced from cytosolic citrate), which is a precursor for organic acid production. In combination with the overexpression and/or inhibition of the beforementioned enzymes, ATP-citrate lyase overexpression will lead to improved organic acid production in general and more specifically itaconic acid and/or citramalic acid. Preferably, the increase of level of cytosolic acetyl-CoA is caused by overexpression of ATP-citrate lyase, more particularly An11g00510 and/or An11g00530

The genes encoding enzyme(s) to be overexpressed preferably is/are expressed under the control of a strong constitutive promoter e.g. PgpdA from *Aspergillus.* Recombinant host cells can be obtained using methods known in the art for providing cells with recombinant nucleic acids. These include transformation, transconjugation, transfection or electrop oration of a host cell with a suitable plasmid (also referred to as vector) comprising the nucleic acid construct of interest operationally coupled to a promoter sequence to drive expression. Host cells of the invention are preferably transformed with a nucleic acid construct as further defined below and may comprise a single but preferably comprises multiple copies of the nucleic acid construct. The nucleic acid construct may be maintained episomally and thus comprise a sequence for autonomous replication, such as an ARS sequence. Suitable episomal nucleic acid constructs may e.g. be based on the yeast 2µ or pKDI (Fleer et al., 1991, Biotechnology 9: 968-975) plasmids. Preferably, however, the nucleic acid construct is integrated in one or more copies into the genome of the host cell. Integration into the host cell's genome may occur at random by illegitimate recombination but preferably the nucleic acid construct is integrated into the host cell's genome by homologous recombination as is well known in the art of fungal molecular genetics (see e.g. WO 90/14423, EP-A-0 481 008, EP-A-0 635 574 and US 6,265, 186). Transformation of host cells with the nucleic acid constructs of the invention and additional genetic modification of the fungal host cells of the invention as described above may be carried out by methods well known in the art. Such methods are e.g. known from standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Pubhshing and Wiley Interscience, New York (1987). Methods for transformation and genetic modification of fungal host cells are known from e.g. EP-A-0 635 574, WO 98/46772, WO 99/60102 and WO 00/37671.

In another aspect the invention relates to a nucleic acid construct comprising a nucleotide sequence encoding the various enzymes or homologs or orthologs thereof as defined above for improved citramalate production and used for transformation of a host cell as defined above. In the nucleic acid construct, the nucleotide sequence encoding such an enzyme preferably is operably linked to a promoter for control and initiation of transcription of the nucleotide sequence in a host cell as defined below. The promoter preferably is capable of causing sufficient expression of the gene encoding theses enzyme in the host cell. Promoters useful in the nucleic acid constructs of the invention include the promoter that in nature provides for expression of these genes. Further, both constitutive and inducible natural promoters as well as engineered promoters can be used. Promoters suitable to drive expression of these genes in the hosts of the invention include e.g. GAL7, GALIO, or GAL 1, CYC1, HIS3, PGL, PH05, ADCI, TRP1, URA3, LEU2, ENO, TPI, and AOX1. Other suitable promoters include PDC, GPD1, PGK1, TEF, TDH, promoters from glycolytic genes (e.g. from a glyceraldehyde-3 -phosphate dehydrogenase gene), ribosomal protein encoding gene promoters, alcohol dehydrogenase promoters (ADH1, ADH4, and the like), promoters from genes encoding amylo- or cellulolytic enzymes (glucoamylase, TAKA- amylase and cellobiohydrolase). Other promoters, both constitutive and inducible and enhancers or upstream activating sequences will be known to those of skill in the art. The promoters used in the nucleic acid constructs of the present invention may be modified, if desired, to affect their control characteristics. Preferably, the promoter used in the nucleic acid construct for expression of the genes is homologous to the host cell in which the genes are expressed.

In the nucleic acid construct, the 3'-end of the nucleotide acid sequence encoding the enzyme preferably is operably linked to a transcription terminator sequence. Preferably the terminator sequence is operable in a host cell of choice. In any case the choice of the terminator is not critical; it may e.g. be from any fungal gene, although terminators may sometimes work if from a non-fungal, eukaryotic, gene. The transcription termination sequence further preferably comprises a polyadenylation signal.

Optionally, a selectable marker may be present in the nucleic acid construct. As used herein, the term "marker" refers to a gene encoding a trait or a phenotype which permits the selection of, or the screening for, a host cell containing the marker. A variety of selectable marker genes are available for use in the transformation of fungi. Suitable markers include auxotrophic marker genes involved in amino acid or nucleotide metabolism, such as e.g. genes encoding ornithine-transcarbamylases (argB), orotidine-5'-decaboxylases (pyrG, URA3) or glutamine-amido-transferase indoleglycerol-phosphate-synthase phosphoribosyl-anthranilate isomerases (trpC), or involved in carbon or nitrogen metabolism, such e.g. niaD or facA, and antibiotic resistance markers such as genes providing resistance against phleomycin, bleomycin or neomycin (G418). Preferably, bidirectional selection markers are used for which both a positive and a negative genetic selection is possible. Examples of such bidirectional markers are the pyrG (URA3), facA and amdS genes. Due to their bidirectionality these markers can be deleted from transformed filamentous fungus while leaving the introduced recombinant DNA molecule in place, in order to obtain fungi that do not contain selectable markers. This essence of this MARKER GENE FREE™ transformation technology is disclosed in EP-A-0 635 574, which is herein incorporated by reference. Of these selectable markers the use of dominant and bidirectional selectable markers such as acetamidase genes like the amdS genes of A. nidulans, A. niger and P. chrysogenum is most preferred. In addition to their bidirectionality these markers provide the advantage that they are dominant selectable markers that, the use of which does not require mutant (auxotrophic) strains, but which can be used directly in wild type strains.

Optional further elements that may be present in the nucleic acid constructs of the invention include, but are not limited to, one or more leader sequences, enhancers, integration factors, and/or reporter genes, intron sequences, centromers, telomere and/or matrix attachment (MAR) sequences. The nucleic acid constructs of the invention may further comprise a sequence for autonomous replication, such as an ARS sequence. Suitable episomal nucleic acid constructs may e.g. be based on the yeast 2µ or pKDI (Fleer et al., 1991, Biotechnology 9: 968-975) plasmids. Alternatively the nucleic acid construct may comprise sequences for integration, preferably by homologous recombination (see e.g. WO98/46772). Such sequences may thus be sequences homologous to the target site for integration in the host cell's genome. The nucleic acid constructs of the invention can be provided in a manner known per se, which generally involves techniques such as restricting and linking nucleic acids/nucleic acid sequences, for which reference is made to the standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987).

Citramalate production can also be improved by inhibition of the citramalic acid downstream pathway, in particular by inhibition of the enzyme citramalate dehydratase (EC4.2.1.34) Preferably, the citramalate dehydratase activity is chosen from genes also encoding aconitate hydratase (e.g. An08g10530, An09g03870, An02g11040, An16g05760), aconitase (e.g. An15g07730), homoaconitate hydratase (e.g. An15g00350) and isopropylmalate dhydratase (e.g. An02g03250).
Further preferably, also inhibition of the enzyme citramalate lyase can lead to improved citramalate levels. Citramalate lyase activity is chosen from genes also encoding oxaloacetate acetylhydrolase (e.g. An07g08390), oxaloacetate hydrolase (e.g. An11g07720), methyl-isocitrate lyase (e.g. An12g07630), isocitrate lyase (e.g. An01g09270), malate synthase (e.g. An15g02980, An12g05180).
Of course inhibition of orthologs and functional homologs of the citramalate hydratase enzymes and citramate lyase enzymes are useful in the present invention when these genes would be present in the fungal strains that are chosen for the production of the citramalic acid.

The genes encoding enzymes to be eliminated and/or repressed preferable are inhibited by inhibition on transcriptional or translational level, e.g. by mutation, anti-sense inhibition or by RNA interference (RNAi). Mutation of the gene coding for the enzyme can be accomplished by site-directed mutagenesis with a mutated nucleotide sequence, which causes aberrant expression of the enzyme or expression of an aberrant enzyme. Such a mutation can comprise, but is not limited to, the following examples:
1) a change in the promoter sequence of the gene, thereby decreasing promoter function. The promoter is usually situated upstream (5') of the coding sequence. In its broader scope, the term "promoter" includes the RNA polymerase binding site as well as regulatory sequence elements located within several hundreds of base pairs, occasionally even further away, from the transcription start site. Such regulatory sequences are, e.g., sequences that are involved in the binding of protein factors that control the effectiveness of transcription initiation in response to physiological conditions. A change in the promoter sequence can be accomplished by, for instance, deletion of the ribosome binding site or by deletion of the TATA-box, which causes loss of recognition or binding of the polymerase enzyme and thus an inhibited formation of mRNA. Alternatively, the promoter can be shortened, or even deleted totally, or replaced by a promoter which is inducible. In the latter case, enzyme product will only be formed after induction of the promoter. Inducible promoters are known to the person skilled in the art. Typically, the factor that binds specifically to an inducible promoter to activate transcription is present in an inactive form which is then directly or indirectly converted to the active form by the inducer. The inducer may be a chemical agent such as a protein, metabolite (sugar, alcohol, *etc.*)*,* a growth regulator, herbicide, or a phenolic compound or a physiological stress imposed directly by heat, salt, wounding, toxic elements *etc.,* or indirectly through the action of a pathogen or disease agent such as a virus. A cell containing an inducible promoter may be exposed to an inducer by externally applying the inducer to the cell such as by spraying, heating, or similar methods. Inducible promoters are known to those familiar with the art and several exist that could conceivably be used to inhibit expression of the enzyme. Inducible promoters suitable for use in accordance with the present invention include, but are not limited to, the heat shock promoter, promoters inducible by the mammalian steroid receptor system and any chemically inducible promoter. Examples of inducible promoters include the inducible 70 kD heat shock promoter of *Drosophila melanogaster* (Wing et al 1989, Mol Gen Genet, 219: 9-16) and the alcohol dehydrogenase promoter which is induced by ethanol. A promoter that is inducible by a simple chemical is particularly useful. Such simple or common chemicals are used in the induction of so-called gene switch promoters. Examples of gene switch promoters include the alcA/alcR gene switch promoter as described in published International Patent Application No. WO 93/21334; the GST promoter, as described in published International Patent Application Nos. WO 90/08826 and WO 93/031294; and the ecdysone switch system as described in published International Patent Application No. WO 96/37609. In such switch systems, the timing of gene expression is controlled by application of an external chemical. The switch chemical may be applied as a spray or vapor to all or part of the transgenic plant or as a root drench. Examples of suitable switch chemicals are provided in the above references describing switch promoter systems. The external chemical stimulus is preferably an chemical, the use of which is not detrimental to the microbial cells. Inducible switch promoter systems preferably include one or two component systems; nevertheless, systems comprising more than two components are encompassed by the present invention. The alcA/alcR switch promoter system is particularly preferred. In the alcA/alcR promoter switch system, the preferred chemical inducer is ethanol, in either liquid or vapour form. One of the main advantages of the use of ethanol is that small quantities of ethanol generate high levels of expression. The alcA/alcR inducible promoter system is a two-component system involving DNA sequences coding for the alcA promoter and the alcR protein, the expression of which is placed under the control of desired promoters. The alcR protein activates the alcA promoter in the presence of an inducer and any gene under the control of the alcA promoter (in this case the gene coding for the enzyme), will therefore be expressed only in the presence of that inducer. With such a system the activity of the gene construct can be limited both by place and by time. Other gene-switch systems and/or inducible promoters are known in the art and would also be equally applicable. An advantage in using such an inducible promoter or gene-switch system is that it may be possible that expression of the enzyme is desired during certain moments of growth of the culture. In that case, during these times, the inducer can be introduced into the culture, resulting in expression of the enzyme. At other moments, no inducer is present and accumulation of itaconic acid will take place.
2) a change in the coding sequence. Such a change can be effected by the insertion, deletion or change of one of more nucleotides in the open reading frame of the gene coding for citramalyl-CoA pyruvate lyase. Such a change should be able to cause a change in the amino acid sequence of the transcribed enzyme. A preferred change-type is causing a frame-shift mutation by inserting or deleting one or two nucleotides. Such a mutation would distort the three-nucleotide codon based information and would cause construction of a completely different sequence of amino acids from the mutation point until a stop codon would be encountered. Generally, such a frame-shift mutation (especially where the mutation is near the 5' end of the gene) yields proteins which do no longer have the biological function of the enzyme encoded by the original gene. Alternatively, a stop codon can be inserted in the gene, which causes termination of the production of the amino acid sequence at that point, which thus results in the production of N-terminally truncated proteins. Also in this case, when the mutation is located near the 5' end of the gene, the resulting truncated protein will no longer have any biologic functionality.
3) introduction of a protein binding site. The insertion of a protein binding site will cause attachment of the corresponding protein (if present), thereby introducing steric hindrance for transcription of the gene. Preferably such a binding site is introduced in front of or in the neighborhood of the start codon (either in the promoter sequence or in the coding sequence) and the presence of an attached protein will hinder the polymerase to start or continue transcription. This again enables a regulatable system, whereby the amount of transcription can be regulated by the amount of protein that is available for binding. Preferred binding sites for such a system are those specific for the transcriptional repressor protein CreA relevant for carbon-catabolite-repression in *Aspergillus species* (Mathieu, M. et al., 2005, Mol. Microbiol. 56(2):535-548; Felenbok, B. et al., 2001, Prog. Nucleic Acid Res. Mol. Biol. 69:149-204; Mathieu, M. and Felenbok, B., 1994, EMBO J. 13(17):4022-4027).
4) a change in gene splicing. A eukaryotic gene typically is present in a structure in which parts having a coding sequence (called 'exons') are interspersed with parts having a non-coding sequence ('introns'). For a correct expression of the eukaryotic gene the whole gene is transcribed into (pre-)mRNA, but then the introns are spliced out of the RNA to result in a final mRNA only having coding sequences (and some regulatory sequences, such as a poly-A tail).
It is also known in especially in various diseases alternative RNA transcripts can be produced from the same genomic region of DNA. These alternative transcripts are generally known as "variants." More specifically, "pre-mRNA variants" are transcripts produced from the same genomic DNA that differ from other transcripts produced from the same genomic DNA in either their start or stop position and contain both intronic and exonic sequence. Upon excision of one or more exon or intron regions, or portions thereof during splicing, pre-mRNA variants produce smaller "mRNA variants." Consequently, mRNA variants are processed pre-mRNA variants and each unique pre-mRNA variant must always produce a unique mRNA variant as a result of splicing. These mRNA variants are also known as "alternative splice variants." If no splicing of the pre-mRNA variant occurs then the pre-mRNA variant is identical to the mRNA variant.

It is also known in in nature such variants can be produced through the use of alternative signals to start or stop transcription and that pre-mRNAs and mRNAs can possess more than one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites.

As used herein, "antisense mechanisms" are all those involving hybridization of a compound with target nucleic acid, wherein the outcome or effect of the hybridization is either target degradation or target occupancy with concomitant stalling of the cellular machinery involving, for example, transcription or splicing.

As used herein, the terms "precursor mRNA" or "pre-mRNA" refer to an immature single strand of messenger ribonucleic acid (mRNA) that contains one or more intervening sequence(s) (introns). Pre-mRNA is transcribed by an RNA polymerase from a DNA template in the cell nucleus and is comprised of alternating sequences of introns and coding regions (exons). Once a pre-mRNA has been completely processed by the splicing out of introns and joining of exons, it is referred to as "messenger RNA" or "mRNA," which is an RNA that is completely devoid of intron sequences. Eukaryotic pre-mRNAs exist only transiently before being fully processed into mRNA. When a pre-mRNA has been properly processed to an mRNA sequence, it is exported out of the nucleus and eventually translated into a protein by ribosomes in the cytoplasm.
As used herein, the terms "splicing" and "(pre-)mRNA processing" refer to the modification of a pre-mRNA following transcription, in which introns are removed and exons are joined. Pre-mRNA splicing involves two sequential biochemical reactions. Both reactions involve the spliceosomal transesterification between RNA nucleotides. In a first reaction, the 2'-OH of a specific branch-point nucleotide within an intron, which is defined during spliceosome assembly, performs a nucleophilic attack on the first nucleotide of the intron at the 5' splice site forming a lariat intermediate. In a second reaction, the 3'-OH of the released 5' exon performs a nucleophilic attack at the last nucleotide of the intron at the 3' splice site thus joining the exons and releasing the intron lariat. Pre-mRNA splicing is regulated by intronic silencer sequence (ISS), exonic silencer sequences (ESS) and terminal stem loop (TSL) sequences.

As used herein, "modulation of splicing" refers to altering the processing of a pre-mRNA transcript such that there is an increase or decrease of one or more splice products, or a change in the ratio of two or more splice products. Modulation of splicing can also refer to altering the processing of a pre-mRNA transcript such that a spliced mRNA molecule contains either a different combination of exons as a result of exon skipping or exon inclusion, a deletion in one or more exons, or additional sequence not normally found in the spliced mRNA (e.g., intron sequence).

As used herein, "splice site" refers to the junction between an exon and an intron in a pre-mRNA (unspliced RNA) molecule (also known as a "splice junction"). A "cryptic splice site" is a splice site that is not typically used but may be used when the usual splice site is blocked or unavailable or when a mutation causes a normally dormant site to become an active splice site. An "aberrant splice site" is a splice site that results from a mutation in the native DNA and pre-mRNA.

Changing the splicing of a gene and thereby producing expression products that are not or only partial functional may be achieved by blocking the splice sites that are needed for a proper expression of the gene. Changing of the splicing may be effected by introducing antisense oligomeric compounds, generally oligonucleotides or oligonucleotide analogs or mimetics, that are capable of interacting with and/or hybridizing to a pre-mRNA thereby modifying gene expression and/or splicing. Oligomeric compounds can be introduced in the form of single-stranded, double-stranded, circular, branched or hairpins and can contain structural elements such as internal or terminal bulges or loops. Oligomeric double-stranded compounds can be two strands hybridized to form double-stranded compounds or a single strand with sufficient self complementarity to allow for hybridization and formation of a fully or partially double-stranded compound. Enzyme-dependent antisense oligonucleotides include forms that are dependent on RNase H activity to degrade target mRNA, and include single-stranded DNA, RNA, and phosphorothioate antisense. Steric blocking antisense oligonucleotides (RNase-H independent antisense) interfere with gene expression or other mRNA-dependent cellular processes by binding to a target sequence of mRNA. Steric blocking antisense includes 2'-0 alkyl antisense oligonucleotides, morpholino antisense oligonucleotides, and tricyclo-DNA antisense oligonucleotides

In the current invention blocking of splicing sites that cause splicing of intron 1 with the help of oligomeric compounds as defined above would lead to the expression of a non-functional protein.

Another embodiment for providing inhibition of the expression orthologs and functional homologs of the genes encoding the enzymes citramalyl-CoA lyase, the citramalate hydratase and citramate lyase is formed by silencing of the expression of the gene. Basically, three methods for silencing are known at this moment and are contemplated in this application: antisense expression, sense co-suppression and RNA-inhibition. However, the invention is not limited to these methods and any other method which causes silencing of the genes coding for the enzyme citramalyl-CoA pyruvate lyase is included.

For antisense expression, a nucleotide sequence coding for said gene, its homologue or variant, or at least a part thereof of 40 nucleotides or more, is put behind a suitable promoter in anti-sense direction. After transcription of this nucleotide sequence an mRNA is produced which is complementary to the mRNA formed through transcription of the endogenous female suppressor gene. It is well proven by now that production of such an anti-sense mRNA is capable of inhibition of the endogenous expression of the gene for which it is complementary. Furthermore, it has been proven that to achieve this effect even sequences with a less than 100% homology are useful. Also antisense mRNA's which are shorter than the endogenous mRNA which they should inhibit can be used. Generally, it is accepted that mRNA sequences of 23 nucleotides or more which have an identity of 70% or more will be capable of generating an inhibitory effect. The principal patent reference is EP 240,208 of Calgene Inc. There is no reason to doubt the operability of antisense technology. It is well-established, used routinely in laboratories around the world and products in which it is used are on the market.

The second approach is commonly called sense co- suppression. This phenomenon occurs when the gene or part of said gene is expressed in its sense direction. Although this kind of expression when full length genes are used most often results in overexpression of the gene, it has been found that in some cases and especially in cases when a sequence shorter than the full length sequence is used, expression of this gene or fragment causes inhibition of the endogenous gene. The principal patent reference on sense co-suppression is EP 465,572 in the name of DNA Plant Technology Inc.

Sense and antisense gene regulation is reviewed by Bird and Ray (Gen. Eng. Reviews 9: 207-221, 1991). Gene silencing can thus be obtained by inserting into the genome of a target organism an extra copy of the target female suppressor gene coding sequence which may comprise either the whole or part or be a truncated sequence and may be in sense or in antisense orientation. Additionally, intron sequences which are obtainable from the genomic gene sequence may be used in the construction of suppression vectors. There have also been reports of gene silencing being achieved within organisms of both the transgene and the endogenous gene where the only sequence identity is within the promoter regions.

The third possible way to silence genes is by using the so-called RNAi technology, which covers all applications in which double-stranded RNAs are used to achieve silencing of an endogenous gene. As has been demonstrated by Fire et al. (Nature, 391: 806-811, 1998) application of a dsRNA of which one strand is at least partly complementary to the endogenously produced mRNA whether produced intracellularly or added extracellularly is extremely capable of inhibiting translation of the mRNA into a protein. It is believed that this phenomenon works through the intermediate production of short stretches of dsRNA (with a length of 23 nucleotides). To achieve production of dsRNA a construct is made harboring both a sense and an antisense nucleotide sequence (together also called an inverted repeat) of at least 19, usually 23 nucleotides or more, of which one is complementary to the endogenous gene which needs to be silenced. The sense and antisense nucleotide sequences can be connected through a spacer nucleotide sequence of any length which allows for a fold back of the formed RNA so that a double stranded RNA is formed by the sense and antisense sequence. The spacer then serves to form the hairpin loop connecting both sense and antisense sequence. The order of the sense and antisense sequence is not important. It is also possible to combine more than one sense-antisense combination in one and the same construct. If the simple form is depicted as: prom - S - spac - AS - term, also the following constructs can be applied: prom - S1 - spac - AS1 - spac - S2 - spac - AS2 - term, or prom - S2 - spac - S1 - spac - AS1 - spac - AS2 - term. Variations in the built up of the construct are possible, as long as the end product of the transcription of said constructs yields one or more dsRNAs. Alternatively, the double stranded structure may be formed by two separate constructs coding for complementary RNA strands, where RNA duplex formation occurs in the cell. In short notation these constructs then look like: prom1-S1-term1 and prom2-AS1-term2. Prom1 and prom2 can be the same or different but should both be constitutive or fruit-specific promoters, term1 and term2 can be the same or different. Both constructs can be introduced into the cell on the same vector, but can also be introduced using two different vectors.

RNA containing nucleotide sequences identical to a portion of the target female suppressor gene are preferred for inhibition. RNA sequences with insertions, deletions and single point mutations relative to the target sequence have also been found effective for inhibition. Thus, sequences with a sequence identity of less than 100% may be used. Sequence identity may be calculated by sequence comparison and alignment algorithms known in the art (see Gribskov and Devereux, Sequence Analysis Primer, Stockton Press, 1991, and references cited therein), for instance by using the Smith-Waterman algorithm as implemented in the BESTFIT software program using default parameters (e.g. University of Wisconsin Computing Group). Thus, the duplex region of the RNA may be defined functionally as a (double stranded) nucleotide sequence that is capable of hybridizing with a portion of the target gene transcript (e.g., 400 mM NaCl, 40 mM PIPES pH 6.4, 1 mM EDTA, 50ºC to 65ºC hybridization for 12-16 hours; followed by washing). The length of the identical nucleotide sequences should be at least 23 nucleotides, but preferably larger: 40, 50, 100, 200, 300 or 400 bases.

As disclosed herein, 100% sequence identity between the inhibiting construct and the target endogenous gene is not required to practice the present invention. Thus the invention has the advantage of being able to tolerate sequence variations that might be expected due to genetic mutation, strain polymorphism or evolutionary divergence.

Thus also included in the invention are constructs having a nucleotide sequence under control of a suitable promoter wherein said nucleotide sequence comprises a part of 40 or more nucleotides in a sense direction, or in an antisense direction or in an inverted repeat form, of the sequence of the gene coding for the enzyme citramalyl-CoA pyruvate lyase, or homologues or variants thereof.

Alternatively, transcription is prevented by means of the expression of a negatively acting transcription factor acting on the target gene promoter. Such negatively acting transcription factor can be natural or artificial. Artificial negatively acting transcription factors can be employed by the overexpression of an engineered polydactyl zinc-finger transcription factor coupled to a general transcription repressor. According to a further embodiment, the interfering with the target gene consists of destabilizing the target gene mRNA, in particular' by means of nucleic acid molecules that are complementary to the target gene mRNA selected from the group consisting of antisense RNA, RNAi molecules. Virus Induced Gene Silencing (VIGS) molecules, co-suppressor molecules, RNA oligonucleotides or DNA oligonucleotides.

In another embodiment the interfering with the target gene consists of inhibiting the target gene expression product. This can be achieved by means of the expression product(s) of one or more dominant negative nucleic acid constructs, overexpression of one or more suppressors which interact with the target gene product, or by means of one or more chemical compounds. Novel ways to introduce site-specific alterations in transcription of an (eukaryotic) gene is by a variation in the recently described CRISPR-Cas genetic engineering, homologous recombination system. (Cong L et al. Science 2013; 339: 819-823; Mali P et al. Science 2013; 339: 823-826; Cho SW et al. Nat Biotechnol 2013; 31: 230-232; Jinek M et al. Elife 2013; 2: e00471). This variation entails the use of a Cas enzyme that is defective in endonuclease activity, but which retains its ability, when co-expressed with a gRNA, to specifically interfere with transcriptional elongation, RNA polymerase binding or transcription factor binding. This system is also indicated as CRISPRi.(Qi LS et al. Cell 2013; 152: 1173-1183; Larson, MH et al 2013, Nature Protocols 8:2180-2196; Amelio, I. and Melino G., 2015, Cell Death & Differentiation, 22: 3-5)

The above-described systems are all systems that act on expression and do not change the underlying genetic sequence of the gene. In that respect these systems are also relatively easy to switch on or switch off at moments when suppression of expression is needed or when suppression of expression is no longer needed. Such a switch can e.g. advantageously be effected by putting the expression of one or all of the components of the silencing system under control of a specific time- or location-restrained promoter.

Next to changes in the expression of the gene, the gene itself may be changed in such a way that no longer a functional protein is expressed. This may be achieved by mutating the gene. The one or more mutations can be introduced randomly by means of one or more chemical compounds and/or physical means and/or by insertion of genetic elements. Suitable chemical compounds are ethyl methanesulfonate, nitrosomethylurea, hydroxylamine, proflavine, N-methyl-N-nitrosoguanidine, N-ethyl-N-nitrosourea, N-methyl-N-nitro" nitrosoguanidine, diethyl sulfate, ethylene imine, sodium azide, formaline, urethane, phenol and ethylene oxide, Physical means that can be used comprise UV-irradiation, fast-neutron exposures X-rays and gamma irradiation. The genetic element is a transposon, T-DNA, or retroviral element.

More efficient and targeted techniques are provided for by so-called site-directed mutagenesis techniques. Many systems for site-directed mutagenesis (SDM) are known to the skilled person, the most notorious being nuclease based SDM systems such as zinc finger nucleases, transcription activator-like effector nucleases (TALENs), and LAGLIDADG homing endonucleases (Curtin, S.J. et al., 2012, The Plant Genome 5:42-50). Another technology for SDM is based on homologous recombination with the target gene. Very recently, the above discussed CRISPR-Cas system has been proven very effective for SDM based on homologous recombination (see e.g. WO2014/144155).

In an embodiment, TALEN (Transcription Activator-like Effector Nuclease) protein or enzyme is used to disrupt or inactivate one or more genes of the citramalic acid production pathways of a cell. In such an embodiment, TALEN (Transcription Activator-like Effector Nuclease) protein or enzyme is used to inhibit gene selected from the group comprising citramalyl-CoA lyase. A TALEN protein is made of a DNA binding domain and a nuclease domain. The DNA binding domain also has 2 parts - the TAL domain that identifies sequences left to the double strand break (DSB) target is termed TAL-L and the TAL domain that identifies sequence right to the DSB target is termed TAL-R. Both TAL-L and TAL-R domains are expressed as fusion protein with the nuclease domain. The natural TAL effector proteins have two domains: an effector domain and a DNA-binding domain. The structure of the DNA-binding domain can be manipulated such that the domain binds specifically to any DNA sequence in the genome. These DNA-binding protein domains can be linked to a customized effector domain such as a nuclease, thus producing a chimeric TALEN (Transcription Activator-like Effector Nuclease) protein. The DNA-binding domain which provides DNA sequence specificity of TALE/TALEN, consists of a variable number of amino acid repeats. Each repeat contains 33-35 amino acids and recognizes a single DNA base pair. The DNA recognition occurs via 2 hypervariable amino acid residues at positions 12 and 13 within each repeat, called Repeat- Variable Di-Residues (RVDs), which are critical for recognizing specific DNA sequences. The RVDs of the repeats in TAL effectors can be varied to create a TAL protein that recognizes a specific target DNA sequence. RVD is specific to a simple cipher like, NI = A, HD = C, NG = T, NN = G or A (Boch, 2009; Moscou, 2009). N, I, H, D, and G represent one letter amino acid codes. The repeats of DNA binding domain are assembled in a TALE expression vector and co- expressed with a nuclease Fokl endonuclease catalytic domain to create TALE nuclease (TALEN). Such TALENs, once expressed in the cell, bind sequence specifically and create a double stranded break which is repaired by Non Homologous End Joining (NHEJ). During such cellular processes, mutations, i.e. either deletions and/or insertions within the gene sequence render nonfunctional protein products.

"Clustered Regularly Interspaced Short Palindromic Repeats" and "CRISPRs", as used interchangeably herein refers to loci containing multiple short direct repeats that are found in the genomes of approximately 40% of sequenced bacteria and 90% of sequenced archaea. The CRISPR system is a microbial nuclease system involved in defense against invading phages and plasmids that provides a form of acquired immunity. The CRISPR loci in microbial hosts contain a combination of CRISPR-associated (Cas) genes as well as non-coding RNA elements capable of programming the specificity of the CRISPR-mediated nucleic acid cleavage. Short segments of foreign DNA, called spacers, are incorporated into the genome between CRISPR repeats, and serve as a 'memory' of past exposures. Cas9 forms a complex with the 3' end of the sgRNA, and the protein-RNA pair recognizes its genomic target by complementary base pairing between the 5' end of the sgRNA sequence and a predefined 20 bp DNA sequence, known as the protospacer. In nature this complex is directed to homologous loci of pathogen DNA via regions encoded within the crRNA, i.e., the protospacers, and protospacer-adjacent motifs (PAMs) within the pathogen genome. The non-coding CRISPR array is transcribed and cleaved within direct repeats into short crRNAs containing individual spacer sequences, which direct Cas nucleases to the target site (protospacer). By simply exchanging the 20 bp recognition sequence of the expressed sgRNA, the Cas9 nuclease can be directed to new genomic targets. CRISPR spacers are used to recognize and silence exogenous genetic elements in a manner analogous to RNAi in eukaryotic organisms.
Three classes of CRISPR systems (Types I, II and III effector systems) are known. The Type II effector system carries out a targeted DNA double-strand break in four sequential steps, using a single effector enzyme, Cas9, to cleave dsDNA. Compared to the Type I and Type III effector systems, which require multiple distinct effectors acting as a complex, the Type II effector system may function in alternative contexts such as eukaryotic cells. The Type II effector system consists of a long pre-crRNA, which is transcribed from the spacer-containing CRISPR locus, the Cas9 protein, and a tracrRNA, which is involved in pre-crRNA processing. The tracrRNAs hybridize to the repeat regions separating the spacers of the pre-crRNA, thus initiating dsRNA cleavage by endogenous RNase III. This cleavage is followed by a second cleavage event within each spacer by Cas9, producing mature crRNAs that remain associated with the tracrRNA and Cas9, forming a Cas9:crRNA-tracrRNA complex.
The Cas9:crRNA-tracrRNA complex unwinds the DNA duplex and searches for sequences matching the crRNA to cleave. Target recognition occurs upon detection of complementarity between a "protospacer" sequence in the target DNA and the remaining spacer sequence in the crRNA. Cas9 mediates cleavage of target DNA if a correct protospacer-adjacent motif (PAM) is also present at the 3 ' end of the protospacer. For protospacer targeting, the sequence must be immediately followed by the protospacer-adjacent motif (PAM), a short sequence recognized by the Cas9 nuclease that is required for DNA cleavage. Different Type II systems have differing PAM requirements. The S. *pyogenes* CRISPR system may have the PAM sequence for this Cas9 (SpCas9) as 5'-NRG-3', where R is either A or G, and characterized the specificity of this system in human cells. A unique capability of the CRISPR/Cas9 system is the straightforward ability to simultaneously target multiple distinct genomic loci by co-expressing a single Cas9 protein with two or more sgRNAs. For example, the *Streptococcus pyogenes* Type II system naturally prefers to use an "NGG" sequence, where "N" can be any nucleotide, but also accepts other PAM sequences, such as "NAG" in engineered systems (Hsu et al, Nature Biotechnology (2013) doi: 10.1038/nbt.2647). Similarly, the Cas9 derived from *Neisseria meningitidis* (NmCas9) normally has a native PAM of NNNNGATT, but has activity across a variety of PAMs, including a highly degenerate NNNNGNNN PAM (Esvelt et al. Nature Methods (2013) doi: 10.1038/nmeth.2681).
An engineered form of the Type II effector system of *Streptococcus pyogenes* was shown to function in eukaryotic cells for genome engineering. In this system, the Cas9 protein was directed to genomic target sites by a synthetically reconstituted "guide RNA" ("gRNA", also used interchangeably herein as a chimeric single guide RNA ("sgRNA")), which is a crRNA- tracrRNA fusion that obviates the need for RNase III and crRNA processing in general.
In the present invention CRISPR/Cas9-based engineered systems may be used in genome editing of the target organism. The CRISPR/Cas9-based engineered systems may be designed to target any gene, but for the use in the present invention especially a gene selected from the group consisting of citramalyl-CoA pyruvate lyase. The CRISPR/Cas9- based systems may include a Cas9 protein or Cas9 fusion protein and at least one gRNA. The Cas9 fusion protein may, for example, include a domain that has a different activity that what is endogenous to Cas9, such as a transactivation domain.

The CRISPR/Cas9-based system may include a Cas9 protein or a Cas9 fusion protein. Cas9 protein is an endonuclease that cleaves nucleic acid and is encoded by the CRISPR loci and is involved in the Type II CRISPR system. The Cas9 protein may be from any bacterial or archaea species, such as *Streptococcus pyogenes.* The Cas9 protein may be mutated so that the nuclease activity is inactivated. An inactivated Cas9 protein from *Streptococcus pyogenes* (iCas9, also referred to as "dCas9") with no endonuclease activity has been recently targeted to genes in bacteria, yeast, and human cells by gRNAs to silence gene expression through steric hindrance. As used herein, "iCas9" and "dCas9" both refer to a Cas9 protein that has the amino acid substitutions D10A and H840A and has its nuclease activity inactivated. The CRISPR/Cas9-based system may alternatively include a Cas fusion protein. The fusion protein may comprise two heterologous polypeptide domains, wherein the first polypeptide domain comprises a Cas protein and the second polypeptide domain has nuclease activity that is different from the nuclease activity of the Cas9 protein. The fusion protein may include a Cas9 protein or a mutated Cas9 protein, as described above, fused to a second polypeptide domain that has nuclease activity. A nuclease, or a protein having nuclease activity, is an enzyme capable of cleaving the phosphodiester bonds between the nucleotide subunits of nucleic acids. Nucleases are usually further divided into endonucleases and exonucleases, although some of the enzymes may fall in both categories. Well known nucleases are deoxyribonuclease and ribonuclease.

The gRNA provides the targeting of the CRISPR/Cas9-based system. The gRNA is a fusion of two noncoding RNAs: a crRNA and a tracrRNA. The sgRNA may target any desired DNA sequence by exchanging the sequence encoding a 20 bp protospacer which confers targeting specificity through complementary base pairing with the desired DNA target. gRNA mimics the naturally occurring crRNA:tracrRNA duplex involved in the Type II effector system. This duplex, which may include, for example, a 42-nucleotide crRNA and a 75 -nucleotide tracrRNA, acts as a guide for the Cas9 to cleave the target nucleic acid. The "target region", "target sequence" or "protospacer" as used interchangeably herein refers to the region of the target gene to which the CRISPR/Cas9-based system targets. In the present invention this would be a target region in a gene selected from the group consisting of citramalyl-CoA pyruvate lyase. The CRISPR/Cas9-based system may include at least one gRNA, wherein the gRNAs target different DNA sequences. The target DNA sequences may be overlapping. The target sequence or protospacer is followed by a PAM sequence at the 3 ' end of the protospacer. Different Type II systems have differing PAM requirements. For example, the *Streptococcus pyogenes* Type II system uses an "NGG" sequence, where "N" can be any nucleotide.
The gRNA may target any nucleic acid sequence such as the genes mentioned above. The CRISPR/Cas9-based system may use gRNA of varying sequences and lengths. The gRNA may comprise a complementary polynucleotide sequence of the target DNA sequence followed by a PAM sequence. The gRNA may comprise a "G" at the 5' end of the complementary polynucleotide sequence. The gRNA may comprise at least a 10 base pair, at least an II base pair, at least a 12 base pair, at least a 13 base pair, at least a 14 base pair, at least a 15 base pair, at least a 16 base pair, at least a 17 base pair, at least an 18 base pair, at least a 19 base pair, at least a 20 base pair, at least a 21 base pair, at least a 22 base pair, at least a 23 base pair, at least a 24 base pair, at least a 25 base pair, at least a 30 base pair, or at least a 35 base pair complementary polynucleotide sequence of the target DNA sequence, wherein said target sequence is derived from the coding sequence of a gene selected from the group consisting of citramalyl-CoA pyruvate lyase, followed by a PAM sequence. The PAM sequence may be "NGG", where "N" can be any nucleotide. The gRNA may target at least one of the promoter region, the enhancer region or the transcribed region of the target gene.

Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, Wis.) in the "BestFit" utility application. The default parameters for this method are described in the Wisconsin Sequence Analysis Package Program Manual, Version 8 (1995) (available from Genetics Computer Group, Madison, Wis.). A preferred method of establishing percent identity in the context of the present disclosure is to use the MPSRCH package of programs copyrighted by the University of Edinburgh, developed by John F. Collins and Shane S. Sturrok, and distributed by IntelliGenetics, Inc. (Mountain View, Calif.). From this suite of packages the Smith-Waterman algorithm can be employed where default parameters are used for the scoring table (for example, gap open penalty of 12, gap extension penalty of one, and a gap of six). From the data generated the "Match" value reflects sequence identity. Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+Swiss protein+Spupdate+PIR. Details of these programs can be found at the following internet address: http://www.ncbi.nlm.gov/cgi-bin/BLAST. With respect to sequences described herein, the range of desired degrees of sequence identity is approximately 80% to 100% and any integer value there between. Typically the percent identities between sequences are at least 70-75%, preferably 80-82%, more preferably 85-90%, even more preferably 92%, still more preferably 95%, and most preferably 98% sequence identity.

Alternatively, the degree of sequence similarity between polynucleotides can be determined by hybridization of polynucleotides under conditions that allow formation of stable duplexes between homologous regions, followed by digestion with single-stranded-specific nuclease(s), and size determination of the digested fragments. Two nucleic acid, or two polypeptide sequences are substantially homologous to each other when the sequences exhibit at least about 70%-75%, preferably 80%-82%, more preferably 85%-90%, even more preferably 92%, still more preferably 95%, and most preferably 98% sequence identity over a defined length of the molecules, as determined using the methods above. As used herein, substantially homologous also refers to sequences showing complete identity to a specified DNA or polypeptide sequence. DNA sequences that are substantially homologous can be identified in a Southern hybridization experiment under, for example, stringent conditions, as defined for that particular system. Defining appropriate hybridization conditions is within the skill of the art. See, e.g., Sambrook et al., supra; Nucleic Acid Hybridization: A Practical Approach, editors B. D. Hames and S. J. Higgins, (1985) Oxford; Washington, D.C.; IRL Press).

Selective hybridization of two nucleic acid fragments can be determined as follows. The degree of sequence identity between two nucleic acid molecules affects the efficiency and strength of hybridization events between such molecules. A partially identical nucleic acid sequence will at least partially inhibit the hybridization of a completely identical sequence to a target molecule. Inhibition of hybridization of the completely identical sequence can be assessed using hybridization assays that are well known in the art (e.g., Southern (DNA) blot, Northern (RNA) blot, solution hybridization, or the like, see Sambrook, et al., Molecular Cloning: A Laboratory Manual, Second Edition, (1989) Cold Spring Harbor, N.Y.). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

The (recombinant DNA) constructs for use in the methods according to the present invention may be constructed using recombinant DNA technology well known to persons skilled in the art. The recombinant gene constructs may be inserted into vectors, which may be commercially available, suitable for introducing into micro-organisms and suitable for expression of the gene product in the transformed cells.

Selectable markers, which may be included as a part of the introduced recombinant DNA, are used to select transformed or transfected cells (those containing recombinant DNA) over untransformed cells. Examples of suitable markers include genes that provide antibiotic resistance. Cells containing the recombinant DNA are capable of surviving in the presence of antibiotic concentrations that kill untransformed/untransfected cells.

The target organism is a micro-organism which is capable of producing organic acid in general and more specifically itaconic acid and/or citramalic acid. This property may be endogenous, or it may be introduced into the cell by recombinant genetic technologies. Examples of endogenously itaconic producing cells are cells of *Aspergillus terreus,* more particularly *A*. *terreus* strain TN484-M1 (high yield) and strain CM85J (low yield) (Dwiarti, L. et al., 2002, J. Biosci. Bioeng. 1:29-33), strain NRRL 1960 (Riscaldati, E. et al., 2000, J. Biotechnol. 83(3):219-230; Bonarme, P. et al., 1995, J. Bacteriol. 177(12):3573-3578) and strain L.S.H.T.M. Cat. No. Am. 1 (Calam, C.T. et al., 1939, Thom. J. Biochem. 33:1488-1495) and of *A*. *itaconicus* (Kinoshita, 1931, Bot. Mag. 45:30) *Ustilago maydis* (WO 2015/140314; Geiser E,, et al., 2016, Microb Biotechnol. (9)1:116-126) and *Aspergillus oryzae* (Jimenez-Quero, A. et al., 2016, J Microbiol Biotechnol., doi: 10.4014/jmb.1603.03073). For citramalic acid endogeneously producing cells are Methanococcus jannasschii (Howell et al. J Bacteriol. 1999 Jan;181(1):331-3). Recombinantly citramalate producing cells are cells from E. coli (e.g. Wu X, Eiteman MA. Biotechnol Bioeng. 2016 Dec;113(12):2670-2675, Webb et al. 2017 Microbiology DOI 10.1099/mic.0.000581)

Alternatively, a non-natural itaconic and/or citramalic acid producing strain can be constructed by the methods described herein preferably using A. *niger.*

The genes coding for the above-mentioned enzymes can be derived from *Aspergillus* and any of the other species mentioned species according to techniques, which are generally known to a person skilled in the art. Also the construction of an expression vector with these genes and the introduction of such a vector into the host organism lies within the skill of the artisans.

Conversion of itaconic acid to citramalic acid has been shown to occur in the pathogenic bacteria *Yersinia pestis* and *Pseudomonas aeruginosa.* This pathway consists of three steps which convert itaconic acid to citramalic acid. Itaconic acid is converted to itaconyl-CoA by the action of itaconyl-CoA transferase (ICT). Itaconyl-CoA is subsequently converted to citramalyl-CoA by the action of itaconyl-CoA hydratase (ICH) and citramalyl-CoA is converted by itaconyl-CoA transferase (ICT) into citramalic acid (Fig. 1). Alternatively citramalyl CoA can also be converted to acetyl-CoA and pyruvate by citramalyl-CoA lyase (CCL)

Increased production of citramalate can thus be obtained by overexpression of the genes encoding the ICT and ICH genes. As is extensively described herein, overexpression of a gene can be achieved in many different ways. Of course, the overexpression is preferably obtained for one or more of these enzymes as they occur in the itaconic and/or citramalic acid producing micro-organism.

Micro-organisms used in the invention are preferably micro-organisms that naturally produce itaconic acid, citramalic acid and/or citric acid. Preferably overexpression of the genes encoding the above described protein(s) and enzyme(s) is accomplished in filamentous fungi, yeasts and/or bacteria, such as, but not limited to *Aspergillus* sp., such as the fungi *A. terreus, A. itaconicus* and *A*. *niger, Aspergillus nidulans, Aspergillus oryzae or Aspergillus fumigatus, Ustilago zeae, Ustilago maydis, Ustilago sp., Candida sp., Yarrowia lipolytica, Rhodotorula sp.* and *Pseudozyma antarctica,* the bacterium *E. coli* and the yeast *Saccharomyces cerevisiae.* Especially preferred are heterologous itaconic , citramalic and citric acid producing organisms for which the substrates of the biosynthesis pathway are available in the host organism.

Thus, micro-organisms which are known to produce itaconic acid would be suitable candidates for the production of citramalic acid. Such micro-organisms include *Aspergillus* sp., such as the fungi *A. terreus, A. itaconicus* and *A*. *niger, Aspergillus nidulans, Aspergillus oryzae or Aspergillus fumigatus, Ustilago zeae, Ustilago maydis, Ustilago sp., Candida sp., Yarrowia lipolytica, Rhodotorula sp.* and *Pseudozyma antarctica,* the bacterium *E. coli* and the yeast *Saccharomyces cerevisiae.*

It further is contemplated that a heterologous organism, which in nature does not or hardly produce itaconic and/or citramalic acid like *Aspergillus niger* or *Aspergillus oryzae* can be used when providing such an organism with a functional pathway for production of itaconic acid or citramalic acid, by overexpression of the above mentioned genes.

A functional pathway for the production of citramalic acid can be produced by transforming the organisms with the gene coding for cis-aconitate decarboxylase CAD (EC 4.1.1.6) such as the enzyme encoded by the nucleic acid sequence of ATEG_09971.1 and enzymes with similar activities (see EP07112895). The organism can then be further equipped with genes that influence the transport of the metabolic products, such as di/tricarboxylate transporters, capable of transporting, among others, cis-aconitate, citrate or isocitrate from the mitochondrion to the cytosol, preferably the gene encoded by the nucleic acid sequence of ATEG_09970.1. These subsequent processes will lead to an increase in cis-aconitate in the cytosol, which can be further converted to itaconic acid and citramalic acid, using overexpression of the gene encoding the enzyme CAD, (over)expression of genes that influence the transport of the metabolic products, such as di/tricarboxylate transporters, capable of transporting, among others, cis-aconitate, citrate or isocitrate from the mitochondrion to the cytosol, preferably the gene encoded by the nucleic acid sequence of ATEG_09970.1. and the above-mentioned citramalate pathway genes encoding itaconyl CoA transferase and itaconyl CoA hydratase (citramalyl CoA hydrolyase) Also optionally such organisms may also comprise an enzyme that is able to transport itaconic acid or itaconate over the cell membrane, such as the enzyme coded by the nucleic acid sequence of ATEG_09972.1.

Even further optimisation of the present invention can be achieved by modulating the activity of the regulator protein that comprises a zinc finger and a fungal specific transcription factor domain as can be found on the gene cluster that also comprises ATEG_09971.1, wherein this regulator protein is indicated as ATEG_09969.1.

Micro-organisms overexpressing the itaconate synthesizing enzymes, methods to provide such micro-organisms and the sequence information of the genes from the ATEG cluster have been extensively described in the applications WO 2009/014437, WO 2009/104958 and WO 2009/110796, which are hereby incorporated by reference.

The above described processes alone or in combination lead to a subsequent increase of itaconic acid. The combination of improved itaconic acid production and overexpression of the enzymes itaconyl-CoA transferase (EC 2.8.3.-) and/or itaconyl-CoA hydro-lyase (citramalyl-CoA hydrolyase EC4.2.1.56) will give rise to a higher concentration of citramalic acid simultaneously relieving toxic effects that are attributed to this higher amount of the itaconic acid, both inside the cell as well as excreted to the culture medium.

Another functional pathway for the production of citramalic acid can be produced by transforming the organisms with the gene coding for citramalate synthase as described above.

Also optionally such organisms may comprise a cellular transporter that is able to transport citramalate over the cell membrane, such as the transporter coded by the nucleic acid sequence of MfsB (CAK40107.1) as described above.

A further aspect of the invention is related to reduced degradation of citramalic acid precursors by inhibition of the expression of citramalate dehydratase (EC 4.2.1.34) or citramalyl CoA lyase (EC 4.1.3.25) in a host strain producing citramalic acid.

A further aspect of the invention is related to an alternative pathway towards citramalic acid production by overexpression of citramalate synthase and/or Isopropyl malate synthase IPMS (EC2.3.1.182).

A further aspect of the invention is related to improved production of precursor molecules for (the production of) organic acids in general and more specifically for itaconic acid and citramalic acid by overexpression of the genes encoding ATP-citrate lyase (EC2.3.3.8).

Key in the biosynthetic pathway for itaconic and citramalic acid is the localisation of the various substrates. It is thought that production of itaconic and citramalic acid mainly occurs in the cytosol. In many biochemical pathways, the end-product is inhibiting its own production to prevent excess end-product in the biological system. Excess end-product will not only lead to loss of energy in an economical sense, it can also give rise to unwanted side effects such as toxicity. It is contemplated that by depleting the cell or cell organel of itaconic or citramalic acid the formation of new itaconic or citramalic acid will continue without end-product inhibition, thus giving -in total- an increase yield of itaconic/citramalic acid. This can be established by additionally providing the culturing organism with transporters that transport the itaconic/citramalic acid outside the cell. Such transporters have been shown and exemplified in WO 2009/104958 and WO 2009/110796. The genes and methods that are claimed herein are incorporated in the present application by reference. Alternatively, a novel transporter of this class is exemplified by a major facilitator superfamily protein, MfsB, coexpressed and genetically linked to the A. niger citramalate synthase.

The above described genes are preferably derived from *Aspergillus* sp. like, *Aspergillus terreus, Aspergillus niger, Aspergillus nidulans, Aspergillus oryzae* or *Aspergillus fumigatus.* However, it is also possible to derive the genes from other itaconate (or citramalate) producing micro-organisms such as *Ustilago zeae, Ustilago maydis, Ustilago* sp., *Pseudozyma antarctica, Candida* sp., *Yarrowia lipolytica,* and *Rhodotorula* sp.

Recombinant host cells as described above can be obtained using methods known in the art for providing cells with recombinant nucleic acids. These include transformation, transconjugation, transfection or electroporation of a host cell with a suitable plasmid (also referred to as vector) comprising the nucleic acid construct of interest operationally coupled to a promoter sequence to drive expression. Host cells of the invention are preferably transformed with a nucleic acid construct as further defined below and may comprise a single but preferably comprises multiple copies of the nucleic acid construct. The nucleic acid construct may be maintained episomally and thus comprise a sequence for autonomous replication, such as an ARS sequence. Suitable episomal nucleic acid constructs may e.g. be based on the yeast 2µ or pKD1 (Fleer et al., 1991, Biotechnology 9: 968-975) plasmids. Preferably, however, the nucleic acid construct is integrated in one or more copies into the genome of the host cell. Integration into the host cell's genome may occur at random by illegitimate recombination but preferably the nucleic acid construct is integrated into the host cell's genome by homologous recombination as is well known in the art of fungal molecular genetics (see e.g. WO 90/14423, EP-A-0 481008, EP-A-0 635 574 and US 6,265,186) Most preferably for homologous recombination the ku70Δ/ku80Δ techniques is used as described for instance in WO 02/052026 and Krappmann, 2007, Fungal Biol. Rev. 21:25-29).

Transformation of host cells with the nucleic acid constructs of the invention and additional genetic modification of the fungal host cells of the invention as described above may be carried out by methods well known in the art. Such methods are e.g. known from standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

In a further aspect the invention relates to fermentation processes in which the transformed host cells of the invention are used for the conversion of a substrate into citramalic acid. A preferred fermentation process is an aerobic fermentation process. The fermentation process may either be a submerged or a solid state fermentation process.

In a solid state fermentation process (sometimes referred to as semi-solid state fermentation) the transformed host cells are fermenting on a solid medium that provides anchorage points for the fungus in the absence of any freely flowing substance. The amount of water in the solid medium can be any amount of water. For example, the solid medium could be almost dry, or it could be slushy. A person skilled in the art knows that the terms "solid state fermentation" and "semi-solid state fermentation" are interchangeable. A wide variety of solid state fermentation devices have previously been described (for review see, Larroche et al., "Special Transformation Processes Using Fungal Spores and Immobilized Cells", Adv. Biochem. Eng. Biotech., (1997), Vol 55, pp. 179; Roussos et al., "Zymotis: A large Scale Solid State Fermenter", Applied Biochemistry and Biotechnology, (1993), Vol. 42, pp. 37-52; Smits et al., "Solid-State Fermentation-A Mini Review, 1998), Agro-Food-Industry Hi-Tech, March/April, pp. 29-36). These devices fall within two categories, those categories being static systems and agitated systems. In static systems, the solid media is stationary throughout the fermentation process. Examples of static systems used for solid state fermentation include flasks, petri dishes, trays, fixed bed columns, and ovens. Agitated systems provide a means for mixing the solid media during the fermentation process. One example of an agitated system is a rotating drum (Larroche et al., *supra*)*.* In a submerged fermentation process on the other hand, the transformed fungal host cells are fermenting while being submerged in a liquid medium, usually in a stirred tank fermenter as are well known in the art, although also other types of fermenters such as e.g. airlift-type fermenters may also be applied (see e.g. US 6,746,862).

Preferred in the invention is a submerged fermentation process, which is performed in a fed-batch or repeated (fed-)batch mode. In a fed-batch fermentation there is a continuous input of feed containing a carbon source and/or other relevant nutrients in order to improve itaconic/citramalic acid yields. The input of the feed can, for example, be at a constant rate or when the concentration of a specific substrate or fermentation parameter falls below some set point. In a repeated batch fermentation the culture is harvested at regular time-intervals by stopping the fermentation and retrieving the produced product from the medium. Next to refreshing the medium often also part of the microbial culture is discarded, while the rest is used as a new inoculum for a following batch culture.

It is preferred to use a host cell that naturally would contain the enzymes/transporters of the itaconic acid and citramalic acid pathway as depicted in Figs. 1 and 2, and the enzymes/transporters of the citric acid pathways in the cytosol and mitochondrion. However, if the host would lack one or more of these genes, they can be co-introduced with the above described enzymes and proteins. Such a co-introduction can be performed by placing the nucleotide sequence of such a gene on the same plasmid vector as the above described genes, or on a separate plasmid vector.

Further, since the citramalic acid pathway is located partly in the cytosol and partly in the mitochondrion, it is contemplated that overexpression of the genes/enzymes in either or both of those compartments would be desirable. The person skilled in the art will know how to achieve overexpression in the cytosol or mitochondria by using the appropriate signal sequences.

As is already described above, a further part of the invention is formed by a strain that is 'resistant' to the toxic effects of citramalic acid and/or its metabolites, such as itaconic acid. Such a strain may be obtained through spontaneous mutation and selection of strains growing in the presence of concentrations of itaconic acid that are inhibitory to the non-mutant strains. Among these itaconic acid resistant strains are those strains producing high levels of itaconic acid. In such a way the two strains EE#25 and EE#26 that are deposited under the Budapest Treaty with the Westerdijk Institute in Utrecht (The Netherlands) on 22-07-2016 with accession numbers CBS141661 and CBS141662 have been obtained (Table 1). It is of course also possible to induce mutations by chemical or radiation treatment and selection for high itaconic acid producing strains.

Besides improving itaconic/citramalic acid levels, a modification in the conversion pathways can also affect the influence of intermediate acid sensitivity of an itaconic/citramalic acid producing strain, since organic acid toxicity is often not mediated by the organic acid itself but by the CoA-ester generated in the first step of the conversion pathway. Disruption of CoA transferase activity could result in reduced toxicity effects.

Strains and transformants deposited at are listed in Table 1.

**Table 1: Strains and transformants used.**

| **Strains and transformants** | **CBS deposit number** | **Strain description** | **Reference** |
|---|---|---|---|
| *Aspergillus terreus* | | | |
| NRRL 1960 | CBS 116.46 | IA producing *A*. *terreus* WT strain | - |
| *Aspergillus niger* | | | |
| AB1.13 CAD 10.1 | - | Selected *cadA* transformant (*amdS*+) derived from WT strain AB1.13 (*pyrG-*) | Li et al. BMC Biotechnol. (2012) 12:57 |
| AB1.13 CAD 4.1 | CBS 141653 | Selected *pyrG* transformant derived from AB1.13 CAD 10.1 | Li et al. BMC Biotechnol. (2012) 12:57 |
| AB1.13 CAD MFS 3.9 | CBS 141655 | Selected *mfsA* transformant (*pyrG*+) derived from AB1.13 CAD 10.1 | Li et al. Appl. Microbiol. Biotechnol. (2013) 97:3901-11 |
| AB1.13 #49B | CBS 141657 | Selected *mttA* transformant (*hygB*+) derived from AB1.13 CAD MFS 3.9 | Hossain et al. Microb. Cell Fact. (2016) 15:130 |
| CitB #99 | CBS 141659 | Selected *citB* transformant (*phleo*+) derived from AB1.13 #49B | Hossain et al. Microb. Cell Fact. (2016) 15:130 |
| CitB #113 | CBS 141660 | Selected *citB* transformant (*phleo*+) derived from AB1.13 #49B | Hossain et al. Microb. Cell Fact. (2016) 15:130 |
| AB1.13 CAD CitB MMT B5 | CBS 143860 | Selected *mttA* transformant (marker?) derived from AB1.13 CAD CitB #53 | Hossain et al. unpublished results |
| EE #25 | CBS 141661 | Laboratory evolved mutant of CitB #99 able to grow at high IA concentrations | Hossain et al. unpublished results |
| EE#26 | CBS 141662 | Laboratory evolved mutant of CitB #99 able to grow at high IA concentrations | Hossain et al. unpublished results |
| CitB #99 pyrE #67 | CBS 143054 | Selected *pyrE* mutant derived from CitB #99 | Hossain et al. unpublished results |
| CitB #99 ΔICT #RD1 | CBS 143055 | *ictA* (An07g00760) deletion strain (*pyrE*+) of CitB #99 pyrE #67 | Hossain et al. unpublished results |
| CitB #99 ΔICH #RB2 | CBS 143056 | *ichA* (An07g09220) deletion strain (*pyrE*+) of CitB #99 pyrE #67 | Hossain et al. unpublished results |
| AB1.13 cimA B3 | | Selected *cimA* overexpressing transformant derived from AB1.13 | Hossain et al. unpublished results |
| AB1.13 cimA D11 G | CBS 143861 | Selected *cimA* overexpressing transformant derived from AB1.13 | Hossain et al. unpublished results |
| CitB#99-acl G1 Z | CBS 143863 | Selected *acl1* and *acl2* overexpressing transformant derived from CitB#99 pyrE#67 | Hossain et al. unpublished results |

### EXAMPLES

### Improved production of citramalic acid by biochemical conversion of itaconic acid and direct citramalic acid synthesis from acetyl-CoA and pyruvate, as well as improved production of organic acid-precursor acetyl-CoA

### Example 1: Citramalate production in stationary phase itaconic acid (IA) producing strains

*A. niger* controlled batch-cultivations of were performed with IA producing *A*. *niger* strains CitB #99 (CBS 141659), EE #25 (CBS 141661) and AB1.13 CAD CitB MMT B5 (CBS 143860), on 5L scale benchtop New Brunswick Scientific fermenters (BioFlo 3000) at 33ºC. Starting pH was 3.5 after inoculation and medium was allowed to naturally acidify till pH 2.3 and then kept at pH 2.3 by addition of 4M KOH. Dissolved oxygen (DO) tension was 25% at moment of inoculation and DO dropped till 20% and kept at 20%. The system was calibrated with 100% sterile air as 100% DO and 100% N2 as 0% DO. The fermenter was inoculated by 72h old 100 mL baffled shakeflask cultures containing 1.0*10^{∧}8 spores. Medium composition for fermentation and pre-culture (M12+Cu) is listed in Table 2.

**Table 2: Composition of Medium 12+Cu, which is used as production medium for IA. Adapted from An Li et al., (2012).**

| **Component** | **Final concentration (g/l)** |
|---|---|
| NH₄SO₄((NH₄)₂SO₄) | 2.36 |
| KH₂PO₄ | 0.11 |
| MgSO₄ * 7 H₂O | 0.5 |
| CuSO₄ * 5 H₂O | 0.005 |
| FeIISO₄ * 7 H₂O | 0.0006 |
| ZnSO₄* 7 H₂O | 0.0006 |
| NaCl | 0.074 |
| CaCl₂ * 2 H₂O | 0.13 |
| Glucose | 100 |

Cultivations were followed for organic acid production using HPLC analysis using a WATERS e2695 Separations Module equipped with an Aminex HPX-87H column (Bio-Rad) and 5 mM H2SO4 as eluent. Detection of peaks occurred simultaneously by a refractive index detector (WATERS 2414) and a dual-wavelength detector (WATERS UV/Vis 2489). Data processing was done with Empower Pro software (Empower 2 Software, copyright 2005-2008, Waters Corporation, Milford, Massachusetts, USA).

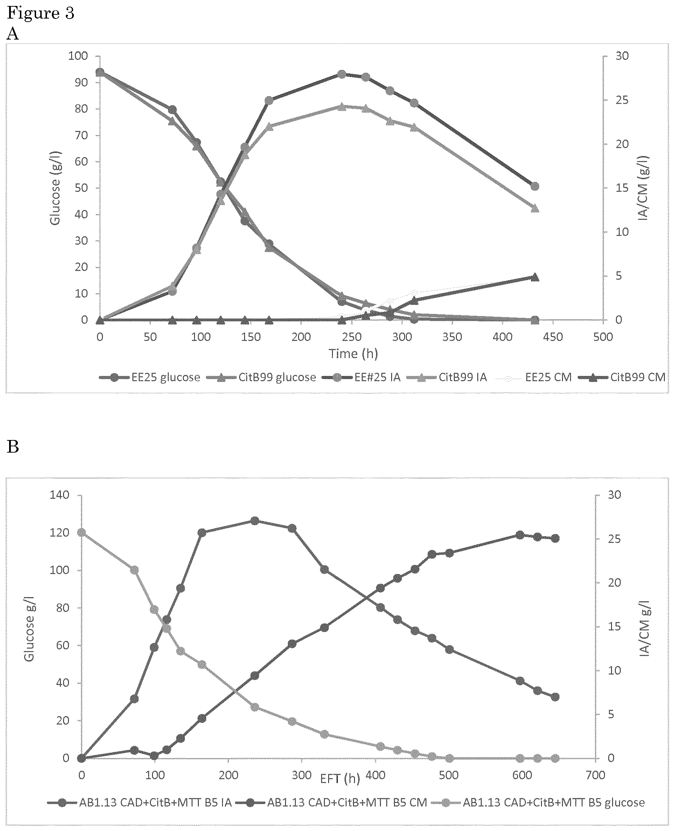

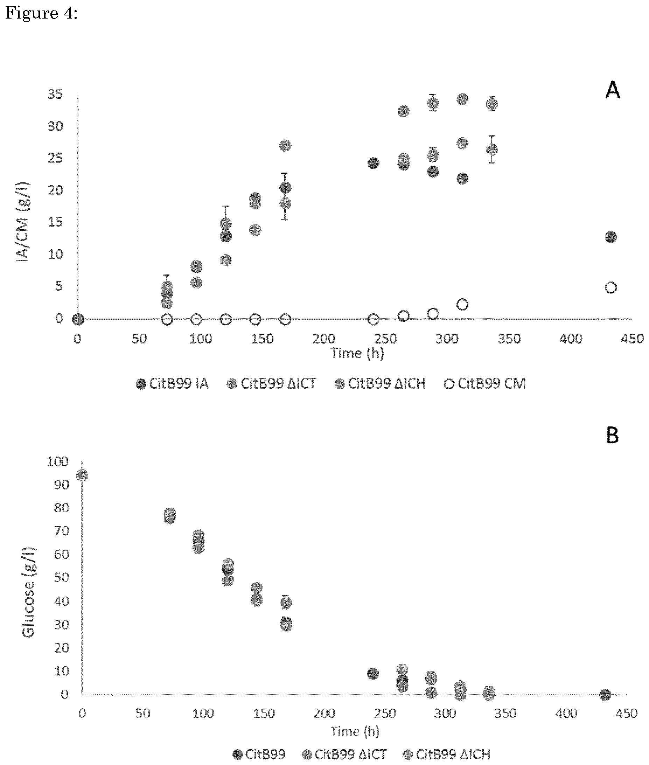

As can be seen in Fig 3 and Fig 4 after prolonged cultivation for all selected strains IA levels declined, concomitant with an increase in citramalic acid (CM) levels.

### Example 2: Transcriptome analysis of high IA producing strains show increased expression of a new organic acid pathway

Previously IA production of *A*. *niger* strain CitB#99 has been shown to reach high IA titers by expressing the *A*. *terreus* biosynthetic IA pathway in combination with an *A*. *niger* citrate synthase without mitochondrial targeting sequences (Hossain et al., 2016). To understand the effect of heterologous IA production on *A*. *niger* metabolism we have analysed a transcriptome dataset from RNA isolated from biomass from different timepoints in shake flask cultures and controlled batch fermentations from low, medium and high IA producing *A*. *niger* strains.
Controlled batch-cultivations were performed on 5L scale benchtop New Brunswick Scientific fermenters (BioFlo 3000) at 33°C. Starting pH was 3.5 after inoculation and medium was allowed to naturally acidify till pH 2.3 and then kept at pH 2.3 by addition of 4M KOH. Dissolved oxygen (DO) tension was 25% at moment of inoculation and DO dropped till 20% and kept at 20%. The system was calibrated with 100% sterile air as 100% DO and 100% N₂ as 0% DO. The fermenter was inoculated by 72h old 100 mL baffled shakeflask cultures containing 1.0*10^8 spores. Medium composition for fermentation and pre-culture (M12+Cu) are listed in Table 2 Li, A., Pfelzer, N., Zuijderwijk, R., and Punt, P. (2012). Enhanced itaconic acid production in Aspergillus niger using genetic modification and medium optimization. BMC Biotechnol. *12*, 57.]. Biomass samples for RNA isolation were taken at several timepoints during fermentation and washed with distilled water and frozen in liquid N₂. The mycelium was disrupted by bead-beating with 0.1 mm acid-washed Zirconium-Silica beads and RNA extraction proceeded using the ChargeSwitch RNA extraction protocol from Invitrogen (Carlsbad, CA, USA). Quality control was checked on 1x MOPS/6% Formaldehyde agarose gels and stained with ethidium bromide.
BaseClear in Leiden, NL performed digital gene expression profiling experiments based on RNA-Seq with an Illumina HiSeq 2000 System. Approximately 8-32 M unfiltered paired-end (PE) reads (99bp/read on ∼320bp cDNA inserts) were obtained. Reads were trimmed of the first 2 bases of the 5' end because these bases showed an aberrantly low GC content. The reads were then further filtered, such that all quality phred scores after filtering are at least 22, with a read-length of at least 40 bases. Around 70-80% of the bases passed these criteria (including a 2% loss because of clipping). After filtering the # PE-reads/samples were betweem 7.6M and 19.8M for all the samples respectively.
Reads were aligned to the 20 contigs in a FastA file of the Aspergillus niger reference genome (from http://www.ebi.ac.uk/ena). Source EMBL annotations were converted to GFF format. The embl data appeared to be derived from multiple sources with different feature tags. These were converted to one uniform GFF format that could be accepted by our third-party software (consistent gene_ids across all contigs). Missing gene definitions (e.g. for CAD) were inserted. The reads were aligned to the reference genome using software based on a Burrows-Wheeler Transform (BWT) algorithm. A mismatch rate of 4% was allowed for the alignment. The maximum insertion length was 3. The maximum deletion length was 3. All samples had more than 85% of the reads aligned, resulting in SAM alignment files. Gene expression was measured as the number of aligned reads to reference genes and was normalized to RPKM values (Reads Per Kb per Million reads; Mortazavi, A., Williams, B. a, McCue, K., Schaeffer, L., and Wold, B. (2008). Mapping and quantifying mammalian transcriptomes by RNA-Seq. Nat. Methods 5, 621-628..

Hierarchical clustering analysis of the full data set was carried out to identify other genes that are correlated to IA production in *A*. *niger.* These data were compared to data from transcriptome analysis in *A*. *terreus* (see accession GSE73044; Chen et al, 2016). In *A*. *terreus* expression of itaconic acid degradation pathway genes was induced under conditions of IA degradation (40 g/l IA addition to culture medium, Table 3).

**Table 3: Gene expression analysis in A. terreus**

| **Protein** | **Gene Code** | **Induced Y/N** | **x-fold (max RKPM)** |
|---|---|---|---|
| ***A.terreus*** | | | |
| Itaconyl CoA transferase | ATEG_06299 | induced | 8.2 (130) |
| Itaconyl CoA dehydratase | ATEG_03709 | induced | 2.3 (320) |
| Citramalyl-CoA lyase | ATEG_03186 | induced | 5.4 (150) |

Amoung the genes identified to be co-regulated by the expression of the itaconic acid gene cluster from the genome wide gene expression analysis of *Aspergillus niger* strains expressing a functional itaconic acid pathway also the genes An07g00760 and An07g09220 (Table 4) showing sequence similarity with genes ATEG_06299 and ATEG_03709 that are identified in *Aspergillus terreus* as genes responsible for the biological degradation of itaconic acid (Chen et al., 2016) are highly expressed in high IA producing conditions. These researchers show that (in a heterologous host) the products of genes ATEG_06299 and ATEG_03709 are itaconyl-CoA transferase (IctA) and itaconyl-CoA hydratase (IchA). These together with citramalyl-CoA lyase (CclA) (ATEG-03186) degrade itaconic acid into the cellular building block chemicals pyruvate and acetyl-CoA [Chen et al., 2016].

**Table 4: Gene expression analysis in A. niger IA producing strains**

| **Protein** | **Gene Code** | **Induced Y/N** | **x-fold (max RKPM)** |
|---|---|---|---|
| | | | |
| Putative Itaconyl CoA transferase homologues | An07g00760, | induced | 5-70 (750) |
| | An18g05120, | not induced | < (10) |
| | An11g10300 | not induced | < (20-30) |
| | | | |
| Putative Itaconyl CoA dehydratase homologues | An07g09220 | induced | 4-100 (850) |
| | An17g02190 | not induced | < (20-30) |
| | | | |
| Putative Citramalyl-CoA lyase homologue | An01g08610 | not induced | < (10-20) |

| | | | |
|---|---|---|---|
| <: no significant fold change in RKPM values | | | |

Our results show a route towards citramalate biosynthesis in *Aspergillus niger.* This route follows the conversion of itaconic acid to citramalate by action of the enzymes itaconyl-CoA transferase (IctA) and itaconyl-CoA hydratase (IchA) in *Aspergillus niger.* In high itaconic acid producing strains the expression of ictA and ichA is upregulated by a factor of 5-70 resp. 4-100x. Interestingly, only the first two genes of the hypothetical *A*. *niger* IA degradation pathway are induced in the IA production strains suggesting that CclA does not take part in the itaconic acid degradation pathway in *A*. *niger* while it does in A. terreus (see table 3).

### Example 3: Overexpression of enzymes itaconyl-CoA transferase (IctA) and itaconyl-CoA hydratase (IchA) in Aspergillus niger result in increased citramalic acid levels

The upregulated expression of ictA and ichA in our CitB#99 strain under high IA producing conditions corresponds to our finding of citramalic acid in late time samples during controlled fermentation (Fig 3, 4), indicating that itaconic acid is intracellularly converted during the production phase into citramalic acid (Fig. 1), which affects the final titer and yield. During cultivation of these high itaconic acid producing strains max itaconic acid titer reaches 25-30 g/l and quickly drops thereafter. During this phase of itaconic acid degradation citramalate accumulation occurs. The bioconversion of itaconate to citramalate seems to occur when glucose is depleted (Fig. 3, 4).
As indicated above two genes An07g00760 and An07g09220 represent a CMA pathway which is clearly induced in an IA producing *A*. *niger* transformant strain indicating a role of both pathways in biochemical conversion towards citramalate during controlled fermentation. Moreover, deletion of any of these two genes had a negative effect on the levels of citramalic acid production, further confirming their role in citramalic acid production
Consequently, strains generated to carry additional copies of both pathway genes, An07g00760 and An07g09220, driven by the constitutive PgpdA promoter show increased levels of citramalic acid.
Further improved levels of citramalic acid are also obtained in fungal strains having a functional itaconic acid biosynthetic and degradation pathway, such as *Aspergillus terreus* and *A*. *niger strains* as described by Hossain et al., 2016, by disruption of the gene encoding the protein responsible for final step of the itaconic acid biosynthetic pathway, i.e. the citramalyl-CoA lyase gene,..

### Example 4: Overexpression of a novel biosynthesis pathway results in increased citramalic acid levels

To date no functional gene is annotated to code for citramalate synthase in *Aspergillus* or fungi in general. BLAST analysis indicates that the putative 2-isopropylmalate synthase shows similarity, albeit low at 26%, with citramalate synthase in *Geobactersulfurreducens* (Risso et al., 2008) precluding an annotation as citramalate synthase. However, transcriptome analysis of high, medium and low IA producing strains revealed strong downregulation of a small gene cluster comprising one of the 2-isopropyl malate synthase-like genes, An09g00170, and of the gene An09g00190 that codes for a a putative MFS multidrug transporter in high IA producing conditions (Table 5).
The citramalate synthase in *E.coli* shows homology to 3 genes in A. niger: 1 homocitrate synthase (32% ID) and 2 isopropylmalate synthases (23-25% ID). All three gene products are predicted to be cytosolic.

**Table 5 Gene expression analysis in A. niger IA producing strains. Values between brackets are presented as RPKM values**

| | | | |
|---|---|---|---|
| HCS | An04g06210 | no differential expression(20-100) | **Lysine biosynthesis** |
| IPMS | An01g13160 | no differential expression(30-100) | **Leucine biosynthesis** |
| IPMS | An09g00170 | 10-fold reduced expression in (most) | |
| | | IA overproducing strains (100-250) | |

| **Citramalate transporter** | | | |
|---|---|---|---|
| MFS | An09g00190 | 10-fold reduced expression in (most) | |
| | | IA overproducing strains(100-200) | |

Interestingly the third gene (An09g00170) is the one responding to IA overproduction, together with a clustered MFS transporter. An09g00170 is annotated as a non-canonical 2-isopropylmalate synthase which has no known mitochondrial or peroxisomal targeting sequence and is therefore suggested to be active in the cytosol.
Close homologues to this hypothetical citramalate synthase and transporter gene are only present in *A*. *niger* CBS513.88 and closely related *Aspergillus* strains but in no other fungus, suggesting that it is part of a unique pathway in *A*. *niger.* Interestingly, the best hit of the transporter protein in *Ustilago* is itp1, the transporter present in the itaconic acid gene cluster in *U. maydis,* although this is not a bidirectional best hit. The best hit of An09g00190 to *A*. *terreus* is ATEG_04174 which is also not bidirectional. The best hit of An09g00190 to *Aspergillus niger* is not the *A*. *terreus* mfsA bidirectional best hit EHA19966 (which is not present in *A.niger* CBS513.88)

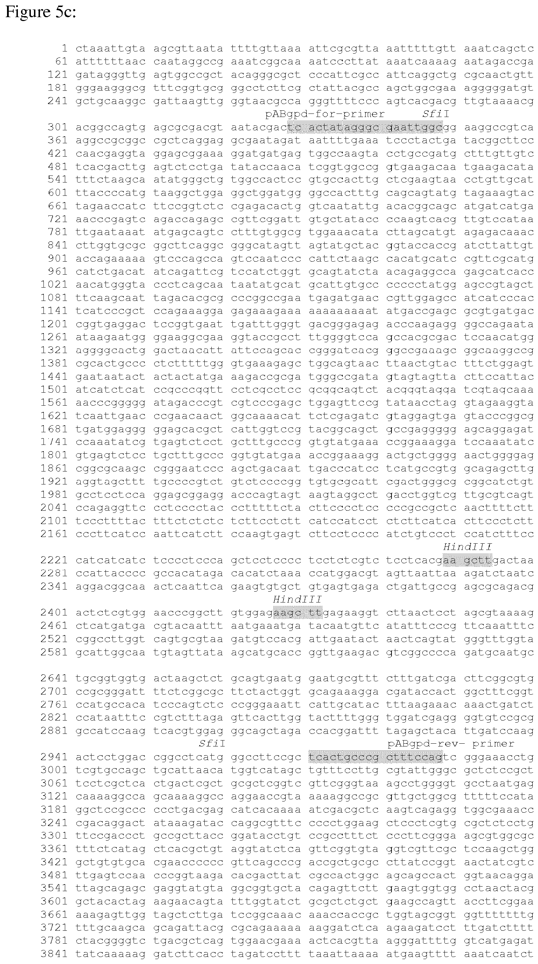

To create an overexpression construct the An09g00170 gene (Fig 5a,b), now termed *cimA* was *in vitro* synthesized at GeneArt (Waltham, MA) and ligated *pABgpdl* vector containing the *A*. *niger gpdA* expression signals (Fig 5c), thereby establishing the *pABgpdl-cimA* expression vector. This construct was co-transformed with pAB4-1, that harbours the *A*. *niger pyrG* gene (van Hartingsveldt et al., 1987), in an ratio of 1:10 (0.5 µg marker: 5 µg construct) into *A*. *niger* AB1.13 (Hossain et al., 2016). All transformations were carried out according to the protocol as reported by Punt, P.J., and Van Den Hondel, C. a M.J.J. (1992). Transformation of filamentous fungi based on Hygromycin B and Phleomycin Resistance Markers. In Methods in Enzymology, pp. 447-457.. Transformed protoplasts were plated on MM agar plates containing sorbitol and grown at 33°C for 3-5 days until colonies were visible. Transformant colonies were purified and analysed by PCR analysis for the presence of introduced copies of the cimA gene. Successful integration of vector pABgpdl-cimA was determined with colony PCR Van Zeijl, C.M.J., Van De Kamp, E.H.M., Punt, P.J., Selten, G.C.M., Hauer, B., Van Gorcom, R.F.M., and Van Den Hondel, C. a M.J.J. (1998). An improved colony-PCR method for filamentous fungi for amplification of pcr-fragments of several kilobases. J. Biotechnol. 59, 221-224. by using the primers pABgpd-for-sfil (TCACTATAGGGCGAATTGGC) and cimA-2,679 R (CGTGAAATCGAACTCGGTCT). PCR positive strains were subsequently cultivated in shakeflask cultures. MM agar plates (16 g/l agar, 6 g/l NaNO₃, 0.52 g/l KCl, 1.52 g/l KH₂PO₄, 10 g/l glucose, 0.0022 g/l ZnSO₄.7H₂O, 0.0011 g/l H₃BO₃, 0.0005 g/l MnCl₂.4H₂O, 0.0005 g/l FeSO₄.7H₂O, 0.00017 g/l CoCl₂.6H₂O, 0.00016 g/l CuSO₄.5H₂O, 0.00015 g/l NaMoO₄.2H₂O, 0.005 g/l Na₂EDTA and 0.5 g/l Mg₂SO₄) were streaked with conidia from glycerol stock or from isolated single colonies. These plates were incubated at 33° C for several days till plates were fully grown. Fresh conidia suspensions were prepared by harvesting conidia from these plates with sterile 0.9% NaCl solution. The harvested conidia were counted on the LUNA II cell counter (Logos Biosystems). Non-baffled shakeflasks (500 mL) were filled with 100 mL M12++ medium and inoculated with 1.0*10⁶/mL conidia. Flasks were weighed when empty, after inoculation and each day before sampling. Evaporation is calculated from the measured weight of the flasks and used to correct measured concentrations of organic acids and glucose on HPLC. As shown in Fig.6 transformants containing the cimA expression vector show increased citamalic acid levels. All three positive transformants produced citramalate whereas the parental AB1.13 strain did not produce citramalate. RNAseq analysis of one of the cimA overexpression strains was carried out confirminfg strong overexpression due to the introduced cimA gene copies (table 6)

**Table 6: Citramalate synthase EC 2.3.1.182 expression in a citramalate overproducing cimA transformant**

| **Protein** | **Gene Code** | **AB1.13 WT RPKM** | **AB1.13 cimA RPKM** |
|---|---|---|---|
| Citramalate synthase (cimA) | An09g00170 | 25 | 4000 |

Strain AB1.13cimA-D11 G was deposited under the Budapest treaty at the Westerdijk Institute under number CBS143861

Based on these results further improved citramalic acid strains were generated by also overexpressing the organic acid transporter coregulated with the cimA gene An09g00190, now termed mfsA. This was done similar to overexpression of cimA using the genomic gene sequences from this gene as shown in Figure 7.

### Example 5: Improved citramalic acid production by reduced expression of citramalic acid bioconversion pathways

To improve final citramalic acid levels in *Aspergillus niger* strains as described in examples 1 and 4 also disruption of citramalate bioconversion pathways was considered. Based on information derived from the KEGG database two different pathways have been considered for the degradation of citramalate to either pyruvate and acetate via a lyase activity or to mesaconate via the dehydratase activity.

Both citramalate dehydratase and citramalate lyase activity are suspected to be involved in citramalate conversion: This activity is found as one of the activities of either aconitater dehydratase/aconitase and isocitrate lyase/oxaloacetate acetyl hydrolase like enzymes (see KEGG database EC4.2.1.34 and EC4.1.3.22). Genome mining was performed for all putative *A*. *niger* homologues of these two gene families (Table 7A, 7B).

**Table 7A: Citramalate dehydratase gene family EC 4.2.1.34**

| **Protein** | **Gene Code** | **Induced Y/N** | **x-fold (max RKPM)** |
|---|---|---|---|
| Aconitate hydratase (AcoA) | An08g10530 | no differential | (200-600) |
| Aconitate hydratase (AcoB) | An09g03870 | no differential | (20-50) |
| | An02g11040 | no differential | (<10) |
| | An16g05760 | no differential | (<10) |
| Aconitase family | An15g07730 | no differential | (20-70) |
| Homoaconitate hydratase (LysF) | An15g00350 | no differential | (20-40) |
| Isopropylmalate dehydratase (LeuCD) | An02g03250 | no differential | (20-100) |

**Table 7B: Citramalate lyase gene family EC=4.1.3.22**

| **Protein** | **Gene Code** | **Induced Y/N** | **x-fold (max RKPM)** |
|---|---|---|---|
| Oxaloacetate acetylhydrolase (OahA) | An10g00820 | 50/100-fold reduced expression in IA overproducing strains(2000-3000) | |
| Oxaloacetate acetylhydrolase | An07g08390 | no differential(5-20) | |
| Oxaloacetate hydrolase like | An11g07720 | no differential(50-300) | |
| Methyl-isocitrate lyase (Mcl) | An12g07630 | no differential(40-80) | |
| Isocitrate lyase (AcuD) | An01g09270 | no differential(30-150) | |
| Malate synthase/isocitrate lyase) | An15g02980 | no differential(20-50) | |
| Malate synthase/isocitrate lyase) | An12g05180 | no differential(<10) | |

Of the 7 dehydratase encoding genes four are closely homologous to proteins of known function in other *Aspergillus* species (name indicated between bracket). Of the remaining, one An15g007730 has likely orthologues in *A*. *terreus* and *A.fumigatus,* An16g05760 has only orthologues in other black *Aspergillus* species like cimA/mfsB does, An02g11040 has likely orthologues is several fungi but not in *A*. *terreus, nidulans* and *fumigatus.* Deletion of any of the three more specific citramalate dehydratase genes, in particular An16g05760 results in reduced citramalate conversion/degradation.

Of the 7 citramalate lyase-like encoding genes three are closely homologous to proteins of known function in other *Aspergillus* species (name indicated between brackets). Of the remaining, one An15g02980 has only orthologues in other black *Aspergillus* species like cimA/mfsB does: An12g05180 and An11g07720 have likely orthologues is several fungi. Deletion of any of the three more specific citramalate lyase genes, in particular An15g02980 results in reduced citramalate conversion/degradation. One of these genes, *oahA* (An10g00820) essential for oxalic acid formation in *A. niger* is very strongly repressed in IA overproducing strains. Overexpression of this gene was analysed in A. *niger* (Kobayashi et al., 2014) but only gives more oxalic acid no citric acid or other acids.

### Example 6: Improved organic acid production by improving pathway precursor levels

The interference of the IA pathway with the "citramalate-pathway" as identified in the present invention runs via acetyl-CoA which is a precursor in both pathways. Therefore, overexpression of **cytosolic** acetyl-CoA via ATP-cityrate lyase was carried out to boost both these pathways. Transcriptome analysis (Table 8) shows that both subunits of cytosolic ATP-citrate lyase are not induced in any IA overproducing strains.

**Table 8 ATP citrate lyase**

| **Protein** | **Gene Code** | **Induced Y/N** | **x-fold (max RKPM)** |
|---|---|---|---|
| ATP-citrate lyase subunit I (AclA) | An11g00510 | no differential(100-350) | |
| ATP-citrate lyase subunit II (AclB) | An11g00530 | no differential(100-400) | |

Based on the annotated gene sequences for aclA and AclB (Fig 8) overexpression vectors were generated based of expression vector pABgpd-1 (Fig 5c) Transformation is performed in both citramalic and itaconic acid overproducing strains.

A selection of transformants is purified and analysed by diagnostic PCR for the disruption of the respective target gene. Correct gene-disruption transformants are identified and used for further research. Controlled fermentation experiments are carried out with several of these strains and their parental host strain showing increased citramalic acid and itaconic acid.

## Claims

1. Method to increase production of citramalic acid in a micro-organism by overexpression of itaconyl-CoA transferase (EC 2.8.3.-), itaconyl-CoA hydratase (citramalyl-CoA hydro-lyase; EC4.2.1.56), and/or citramalate synthase (EC 2.3.1.182).

2. Method according to claim 1, wherein the itaconyl-CoA transferase is ATEG_06299 or An07g00760 or an ortholog thereof.

3. Method according to claim 1 or 2, wherein the itaconyl-CoA hydratase is ATEG_03709 of An07g09220 or an ortholog thereof.

4. Method according to claim 1, 2 or 3, wherein the citramalate synthase is An09g00170 or a protein that has a sequence identity of 60% with the sequence as depicted in Fig. 5b or an ortholog thereof.

5. Method according to claim 4, wherein said protein is coded by a nucleotide sequence as depicted in Fig, 5a.

6. Method to increase production of citramalic acid by inhibition of the citramalic acid bioconversion pathway, in particular by inhibition of the enzyme citramalate dehydratase (EC4.2.1.34) or the enzyme citramalate lyase (EC4.1.3.22), or by an increase in the level of cytosolic acetyl-CoA.

7. Method according to claim 6, wherein the citramalate dehydratase is chosen from aconitate hydratase (e.g. An08g10530, An09g03870, An02g11040, An16g05760), aconitase (e.g. An15g07730), homoaconitate hydratase (e.g. An15g00350) and isopropylmalate dhydratase (e.g. An02g03250).

8. Method according to claim 6, wherein the citramalate lyase is chosen from oxaloacetate acetylhydrolase (e.g. An07g08390), oxaloacetate hydrolase (e.g. An11g07720), methyl-isocitrate lyase (e.g. An12g07630), isocitrate lyase (e.g. An01g09270), malate synthase (e.g. An15g02980, An12g05180)

9. Method according to claim 6, wherein the increase of level of cytosolic acetyl-CoA is caused by overexpression of ATP-citrate lyase, more particularly An11g00510 and/or An11g00530.

10. Method according to any of claims 1-9, wherein said micro-organism is a micro-organism which naturally produces itaconic acid or citramalic acid,.

11. Method according to any of claims 1-10, wherein said micro-organism is genetically constructed to produce citramalic acid, preferably by introducing a gene coding for itaconyl-CoA transferase (EC 2.8.3.-), itaconyl-CoA hydratase (citramalyl-CoA hydro-lyase; EC4.2.1.56), and/or citramalate synthase (EC 2.3.1.182).

12. Method according to any of the above methods, wherein said micro-organism is an *Aspergillus,* preferably *A*. *terreus* or *A*. *niger.*

13. Micro-organism, preferably *Aspergillus,* more preferably *A*. *terreus* or *A*. *niger,* in which expression of the gene(s) coding for the enzyme citramalate dehydratase (EC4.2.1.34) or the enzyme citramalate lyase (EC4.1.3.22) is inhibited.

14. Micro-organism, preferably *Aspergillus,* more preferably *A*. *terreus* or *A*. *niger,* in which the gene(s) coding for the enzyme citramalate synthase (EC 2.3.1.182) is overexpressed.

15. Mutant strains as deposited on 26-01-2018 under no. CBS 143860, CBS 143861 and CBS 143863 with the Westerdijk Institute in Utrecht (The Netherlands)
